(19) Europäisches Patentamt
European Patent Office
Office européen des brevets

(11) **EP 3 196 646 B1**

(12) **EUROPEAN PATENT SPECIFICATION**

(45) Date of publication and mention
of the grant of the patent:
**18.12.2019  Bulletin 2019/51**

(51) Int Cl.:
***G01N 33/68*** (2006.01)

(21) Application number: **16151956.6**

(22) Date of filing: **19.01.2016**

(54) **METHODS OF MAPPING PROTEIN VARIANTS**

VERFAHREN ZUR KARTIERUNG VON PROTEINVARIANTEN

PROCÉDÉS DE MAPPAGE DE VARIANTS DE PROTÉINES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**26.07.2017  Bulletin 2017/30**

(73) Proprietor: **Hexal AG
83607 Holzkirchen (DE)**

(72) Inventors:
• **HIGEL, Fabian
82041 Oberhaching (DE)**
• **SEIDL, Andreas
82041 Oberhaching (DE)**

(74) Representative: **Greiner, Elisabeth
df-mp Dörries Frank-Molnia & Pohlman
Patentanwälte Rechtsanwälte PartG mbB
Theatinerstraße 16
80333 München (DE)**

(56) References cited:
EP-A1- 2 207 030     WO-A1-2010/023471
WO-A1-2014/060551    WO-A2-2004/108948
WO-A2-2006/002841

• **HIGEL FABIAN ET AL: "N-glycosylation
heterogeneity and the influence on structure,
function and pharmacokinetics of monoclonal
antibodies and Fc fusion proteins", EUROPEAN
JOURNAL OF PHARMACEUTICS AND
BIOPHARMACEUTICS, ELSEVIER SCIENCE
PUBLISHERS B.V., AMSTERDAM, NL, vol. 100, 13
January 2016 (2016-01-13), pages 94-100,
XP029395648, ISSN: 0939-6411, DOI:
10.1016/J.EJPB.2016.01.005**
• **HIGEL FABIAN ET AL: "N-glycan PK Profiling
Using a High Sensitivity nanoLCMS Work-Flow
with Heavy Stable Isotope Labeled Internal
Standard and Application to a Preclinical Study
of an IgG1 Biopharmaceutical",
PHARMACEUTICAL RESEARCH, SPRINGER
NEW YORK LLC, US, vol. 32, no. 11, 28 May 2015
(2015-05-28), pages 3649-3659, XP035553879,
ISSN: 0724-8741, DOI:
10.1007/S11095-015-1724-0 [retrieved on
2015-05-28]**
• **Fabian Benjamin Higel: "Dissertation zur
Erlangung des Doktorgrades der
Pharmacokinetic Profiling of Therapeutic
Proteins and Variants by Mass Spectrometry", ,
11 September 2014 (2014-09-11), XP055287203,
Retrieved from the Internet:
URL:https://edoc.ub.uni-muenchen.de/17458/
1/Higel_Fabian_Benjamin.pdf [retrieved on
2016-06-07]**

- P. GILLERY ET AL: "Dosage de l'HbA1c et des produits d'Amadori en biologie humaine - [Assays of HbA(1c) and Amadori products in human biology]", ANNALES PHARMACEUTIQUES FRANÇAISES, vol. 72, no. 5, 6 May 2014 (2014-05-06), pages 330-336, XP55286564, FR ISSN: 0003-4509, DOI: 10.1016/j.pharma.2014.04.002
- SHUANG YANG ET AL: "Glycomic Analysis by Glycoprotein Immobilization for Glycan Extraction and Liquid Chromatography on Microfluidic Chip", ANALYTICAL CHEMISTRY, vol. 85, no. 21, 5 November 2013 (2013-11-05), pages 10117-10125, XP55148101, ISSN: 0003-2700, DOI: 10.1021/ac4013013
- REGNIER F E ET AL: "COMPARATIVE PROTEOMICS BASED ON STABLE ISOTOPE LABELING AND AFFINITY SELECTION", JOURNAL OF MASS SPECTROMETRY, WILEY, CHICHESTER, GB, vol. 37, no. 2, 1 February 2002 (2002-02-01), pages 133-145, XP009023411, ISSN: 1076-5174, DOI: 10.1002/JMS.290

**Description**

*Field of the Invention*

The present invention relates to a method for analysing protein variants of a recombinant protein of interest, such as antibodies or Fc-fusion proteins, in a liquid sample of a mammal. Specifically the method comprises a step of affinity purifying the recombinant protein of interest from the sample together with an internal standard, and analyzing the protein variants using an analytic separating method such as HPLC, capillary electrophoresis or MS. This method is particularly suited to measure pharmacokinetic parameters of a recombinant protein of interest, such as a biopharmaceutical, in a mammal in clinical or pre-clinical studies. It allows for the use of a small sample volume and the possibility to operate with high throughput, such as in a 96-well plate sample preparation. It also provides high sensitivity and allows analysis of protein variants individually.

*Background of the Invention*

Biological drugs belong to the most complex pharmaceuticals. One important class of biological drugs are monoclonal antibodies (mAbs), which are typically glycoproteins with a molecular mass of about 150 kDa. Another important class of biological drugs are fusion proteins, such as Fc-fusion proteins. Biopharmaceuticals, in particular monoclonal antibodies and Fc-fusion proteins are complex mixtures containing several modifications and protein variants (e.g. N-glycosylation, deamidation, isomeration (iso-aspartate), oxidation, N-terminal heterogeneity, C-terminal heterogeneity, disulfide isoform, glycation etc.) These modifications or variants can influence the structure and function of the biopharmaceuticals. In recent years heterogeneity of therapeutic proteins came into focus as several investigations showed that different protein variants may have an effect on the safety and efficacy of the corresponding biological drugs. Further, possible effects of protein variants on the pharmacokinetics (PK) have become of interest. For biopharmaceuticals, especially for biosimilars, it is of vital importance to identify these PK influencing modifications and analysis should be performed already during early biopharmaceutical, particularly during pre-clinical development, when only small sample volumes are available.

During early biopharmaceutical development (clone or pool selection) and during pre-clinical studies, only minute amounts of recombinant protein of interest from micro titer plates are usually available for protein analysis. For studying the influence of protein variants on the pharmacokinetics of biopharmaceuticals, the recombinant protein of interest is further present in a sample, e.g., serum, in admixture with other proteins. Further, the amount of the recombinant protein of interest declines over time. Thus, high sensitivity, specificity and low sample consumption is required. Typically, there is a large sample number, hence the protein of interest must be isolated from the sample in a reproducible manner ideally in a high throughput format and the analysis of the individual samples within a series of samples must be comparable to each other.

The role of glycans in clearing IgG molecules in circulation has been examined by various groups, including pre-clinical or clinical pharmacokinetic (PK) studies, in an attempt to determine the impact of Fc glycan changes on serum clearance. Two general approaches have been used in these studies. In one approach, antibodies have been prepared that are enriched in specific glycan forms, either through genetic mutation, addition of metabolic inhibitors, a combination of both, affinity purification or enzymatic treatment. These antibody samples are then injected into animals to determine the impact on the overall clearance rate. However, this approach has limitations because of the assumption that the only change in the molecule during enrichment is the glycan structure. Glycan heterogeneity can also lead to some ambiguity of the results. Also different variants of the same product need to be prepared and administered. Thus, this approach is not suitable for pre-clinical or clinical pK studies of biopharmaceuticals, such as therapeutic recombinant antibodies or fusion proteins.

In another approach, the glycan forms are analyzed after the drug has been administered and changes to the glycan pattern with circulation time are interpreted as differences in clearance rates. The post-administration collection approach can follow many more glycan forms simultaneously. Since changes to a single administered sample are followed, the impact of specific microheterogeneity can be monitored. To the best of our knowledge this approach has only been used in humans, but not in smaller mammals typically used in pre-clinical studies, where sample size is more limiting. However, analysis of glycans on pharmacokinetics as early as possible in the development of biopharmaceuticals is desirable, as well as reducing the sample size needed in clinical trials.

Alessandri et al (mAbs, 2012, 4(4), 509-520) analyzed a recombinant monoclonal IgG1 antibody from serum samples obtained from a human PK study. The antibody was purified from serum by affinity chromatography using its ligand cross-linked to sepharose beads. The glycans were released from the acidically eluted antibody, labeled with 2-aminobenzamide (2-AB) and analyzed by normal phase high performance liquid chromatography. However, the assay allowed analysis only up to 14 days post-administration in a rather high sample volume and is prone to contamination with other serum proteins.

Chen et al (Glycobiology 2009, 19(3), 240-249) analyzed monoclonal antibodies in serum samples of a pharmacokinetic study in humans. The antibodies were affinity purified using a ligand cross-linked to a resin and acidically eluted thereof. The released glycans were labeled with 2-aminobenzamide (2-AB) and analyzed by RP-HPLC/MS. This isolation method was capable of obtaining only 70% of the specific antibody from the serum. Also, this method is subject to interference from glycans released from other serum proteins or other glycans of the same molecule for glycoproteins with more than one glycosylation sites.

Goetze et al (Glycobiology 2011, 21(7), 949-959) analyzed monoclonal IgG1 and IgG2 antibodies in serum samples of a pharmacokinetic study in humans. The antibodies were affinity purified using the respective ligand cross-linked to a resin. The antibodies were acidically eluted and digested with either the endoproteinase Lys-C or trypsin. The glycosylated peptides were analyzed using LC-MS/MS. This method offers the advantage of being specific for the consensus Fc glycosylation of either human IgG1 or human IgG2. The applied peptide mapping approach reduces the potential pool of interfering impurities to only endogenous IgG of the same subclass as the mAb to be analyzed. However, this method requires a sample volume of 0.5 ml, which often may not be available, e.g., in preclinical studies using rodents. Also this analysis allows only for determining the relative proportion of N-glycan structures within a sample, but not for an independent analysis of single N-glycan structures.

In EP 2 975 401, an efficient and sensitive analysis of neutral and acidic N-glycans by RP-LC-MS for PK profiling was described. N-glycan PK profiling using this high sensitive nanoLCMS work-flow with heavy stable isotope labeled internal standard and its application to a preclinical study of an IgG1 biopharmaceutical was further described by Higel et al (Pharmaceutical Research 2015, 32(11), 3649-59). This method may be used with RP-nano-LC-MS and a 96-well plate sample preparation, which allows attomolar sensitivity and high throughput. However, analysis on modifications other than glycosylation was not performed.

Variants other than glycosylation variants may also influence the pharmacokinetic of a biotherapeutic protein, such as deamidation, isomeration (iso-aspartate), oxidation, N-terminal heterogeneity, C-terminal heterogeneity, disulfide isoform or glycation. Only very few studies in the literature have addressed or measured the influence of these protein variants on the PK of a biotherapeutic protein. Khawali et al (mAbs 2010, 2(6), 613-624) studied IgG1 charge variants in rats. They isolated the major charge forms of a recombinant humanized IgG1 and injected them into animals to determine the impact on the PK. However, this approach has limitations because of the assumption that the only change in the molecule during enrichment is the charge form. Also different variants of the same product need to be prepared and administered. Thus, this approach is not suitable for pre-clinical or clinical pK studies of biopharmaceuticals, such as therapeutic recombinant antibodies or fusion proteins.

Liu et al (JBC 2008, 283(43), 29266-29272) analyzed human IgG2 antibody disulfide rearrangement *in vivo* in human patients. The IgG2 antibody was affinity purified and the different disulfide isoforms analyzed over time by RP-HPLC. However, this method requires a sample volume of 0.5 ml, which often may not be available, e.g., in preclinical studies using rodents. Also this analysis allows only for determining the relative proportion of variants within a sample, but not for an independent analysis of single protein variants. WO 2004/108948 discloses a method for analyzing phosphorylation of polypeptides using a peptide as internal standard.

Thus, there was a need for a method to analyze individual protein variants other than N-glycosylation of a recombinant protein of interest, such as monoclonal antibodies or Fc-fusion proteins in a series of mammalian samples, such as serum, rather than a proportion of protein variants within the sample. There was also a need for high sensitivity to allow for low sample consumption and a sample preparation to be operated in a high throughput manner.

### *Summary of the Invention*

Accordingly, the present invention relates to a method of analyzing protein variants of a recombinant protein of interest in liquid samples of a mammal comprising a) providing two or more liquid samples of a mammal comprising the recombinant protein of interest; b) immobilizing the recombinant protein of interest of each of said samples on a separate solid support coupled to an affinity ligand specific for the recombinant protein of interest in the samples; c) eluting the recombinant protein of interest or a fragment thereof of each of said samples from the solid support into separate eluates; and d) analyzing the protein variants of step c) of each of said samples separately using an analytical separating method and comparing said two or more samples, wherein an internal standard binding to the same affinity ligand is added to each of said samples prior to step b and the internal standard is analyzed together with the recombinant protein of interest of step d), and wherein the protein variants of the recombinant protein of interest to be analyzed are due to deamidation, oxidation, N-terminal heterogeneity, C-terminal heterogeneity, isomerisation, or disulfide isoforms.

The method of the present invention may be used for analyzing one or more pharmacokinetic parameter of said protein variants.

According to the present invention the analytical separating method suitable to distinguish said protein variants of the recombinant protein of interest is preferably selected from the group consisting of HPLC, capillary electrophoresis (CE) or mass spectrometry (MS). MS may further be coupled to HPLC or CE for analysis.

In case the protein variants of the recombinant protein of interest are due to deamidation, oxidation, N-terminal heterogeneity, C-terminal heterogeneity, isomerization or glycation, the analytical separating method is preferably selected from the group consisting of HPLC, CE and MS. In case the protein variants of the recombinant protein of interest are due to disulfide isoforms, the analytical separating method is selected from the group consisting of HPLC and CE. If the analytical separating method is MS it may further be coupled to HPLC or capillary electrophoresis, LC-MS is preferred, and NanoLC-MS is even more preferred;

The internal standard should preferably bind to the affinity ligand with an affinity comparable to the recombinant protein of interest and is distinguishable from the recombinant protein of interest using the suitable analytical separating method.

The recombinant protein of interest is preferably a fusion protein or an antibody, more preferably a Fc-fusion protein or an antibody, wherein the antibody is preferably a monoclonal antibody.

If the recombinant protein of interest is an antibody and the protein variation does not affect binding of said antibody to its ligand, the variable region of the antibody may bind to the affinity ligand immobilized on the solid support, and the affinity ligand is preferably an antigen.

If the recombinant protein of interest is a Fc-fusion protein comprising a Fc-domain and an effector domain, the effector domain may bind to the affinity ligand immobilized on the solid support, and the affinity ligand is a binding partner or an antibody specifically binding to the effector domain of the Fc-fusion protein.

Alternatively, the recombinant protein of interest is a Fc-fusion protein or an antibody and the affinity ligand binds to the Fc domain of the recombinant protein of interest, without binding to endogenous antibodies in the sample.

According to the invention the recombinant protein of interest may be an IgG antibody, preferably an IgG1 or IgG2 antibody, more preferably a human or humanized IgG1 or IgG2 antibody.

The solid support according to the invention is a resin comprising microbeads, preferably sepharose beads, agarose beads or magnetic beads, more preferably magnetic beads. The affinity ligand may be coupled to the solid support via N-hydroxysuccinimide (NHS), cyanogen bromide, epoxy, carbodiimide or thiopropyl, preferably via N-hydroxysuccinimide (NHS).

In one embodiment of the invention the two or more liquid samples of a mammal are prepared for analysis in a multi-well plate, preferably in a 24, 96 or 384 well plate, more preferably in a 96 well plate, preferably a multi-well filter plate.

The liquid samples of a mammal according to the invention are body fluids, preferably selected from the group consisting of serum, plasma, urine, cerebral spinal fluid, amniotic fluid, saliva, sweat, ejaculate, tears, phlegm, vaginal secretion, vaginal wash and colonic wash; preferably the liquid samples are plasma, serum or urine, more preferably plasma or serum. The liquid sample of a mammal may be a human, a monkey, a rodent, a dog, a cat or a pig sample. Preferably it is a non-human sample, such as a monkey, rodent, dog, cat or pig sample. If the sample is a rodent sample it may be a mouse, a rat, a hamster or a rabbit sample.

The methods according to the invention are suitable to determine one or more pharmacokinetic parameters of at least one specific protein variant of the recombinant protein of interest, preferably the $C_{max}$, $t_{max}$, AUC or $t_{1/2}$.

According to the invention, the protein variants may be analyzed in an aliquot of each of said two or more liquid samples of a mammal and optionally the concentration of said recombinant protein of interest is analyzed in a further aliquot of said two or more liquid samples of a mammal.

In one embodiment, the samples or the aliquots to be analyzed have a volume from about 1 $\mu$l to about 1000 $\mu$l, from about 5 $\mu$l to about 500 $\mu$l, from about 10 $\mu$l to about 200 $\mu$l, from about 10 $\mu$l to about 100 $\mu$l, from about 25 $\mu$l to about 100 $\mu$l, or from about 40 $\mu$l to about 75 $\mu$l, preferably of about 50 $\mu$l.

## Brief Description of the Figures

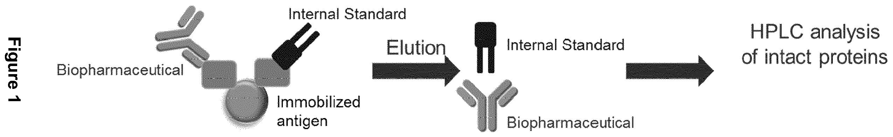

Figure 1: Affinity purification work-flow. Schematic illustration of the work flow of the disulfide PK profiling method exemplified for a monoclonal IgG2 antibody as biopharmaceutical. Antigen immobilized to magnetic beads binds to the biopharmaceutical (monoclonal IgG2 antibody) and a fusion protein binding to the same antigen as internal standard spiked into the serum sample. After washing the complex, the internal standard and biopharmaceutical are eluted and analyzed by reverse phase HPLC.

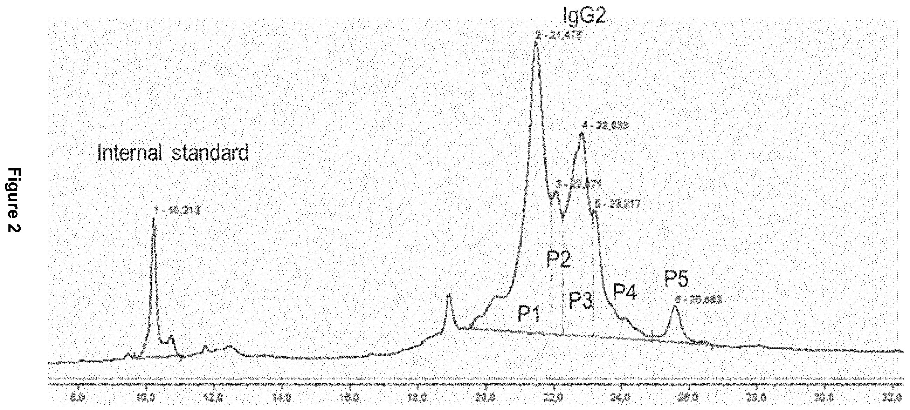

Figure 2: Example reverse phase chromatogram showing the peaks of the internal standard and the peaks of the IgG2 biopharmaceutical. The peaks are shown at 1-10,213 (internal standard), 2-21,475 (P1), 3-22,071 (P2), 4-22,833 (P3), 5-23,217 (P4) and 6-25,583 (P5).

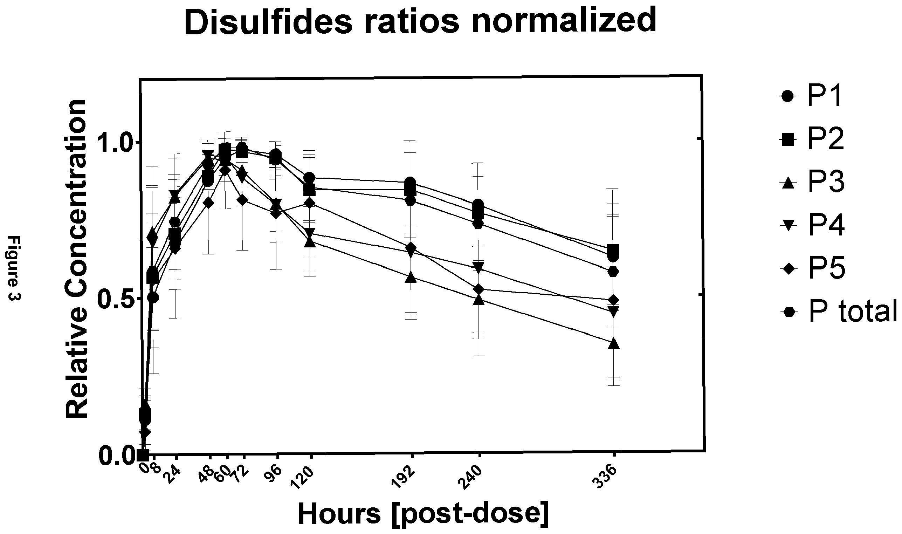

Figure 3: PK profiles of the five different reverse phase chromatography peaks. Results of a preclinical study comparing relative concentrations of disulfide isoforms over time. PK profiles of disulfide variants indicating that P3 to P5 (corresponding to the peaks in Figure 13) have different PK profiles.

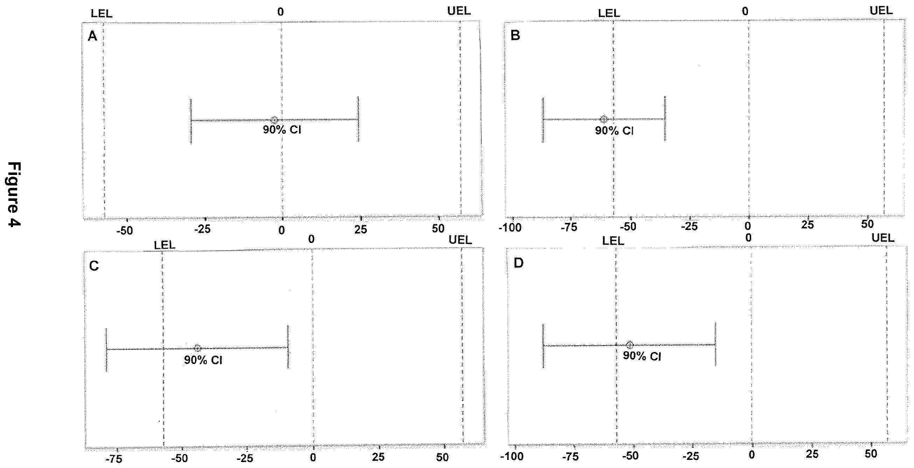

**Figure 4:** Equivalence test results. Equivalence testing of P2, P3, P4 and P5 was performed against the main variant P1 and is shown in (A) for P2, 90% CI for Mean P2 - Mean P1 (-29.058; 24.558) (B) for P3, 90% CI for Mean P3 - Mean P1 (-86.6077; -35.226) (C) for P4, 90% CI for Mean P4 - Mean P1 (-78.902; -9.3984), and (D) for P5, 90% CI for Mean P5 - Mean P1 (-87.727; -78.902). LEL indicates the "Lower Equivalence Limit" and UEL indicates the "Upper Equivalence Limit".

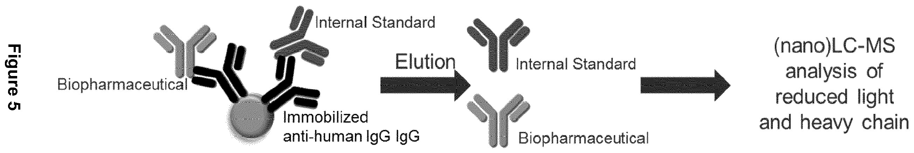

**Figure 5:** Schematic illustration of the work flow of the N-terminal heterogeneity PK profiling method exemplified for a humanized monoclonal IgG1 antibody as biopharmaceutical from a rodent serum sample. Anti-human IgG antibody immobilized to magnetic beads binds to the human IgG1 biopharmaceutical and a control human IgG1 as internal standard spiked into the serum sample. After washing the complex, the internal standard and biopharmaceutical are eluted and the reduced light and heavy chain is analyzed by (nano)LC-MS.

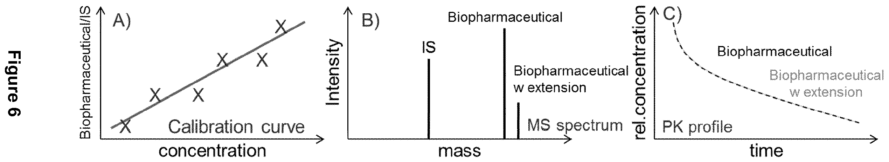

**Figure 6:** (A) Example calibration curve calculated from the human IgG antibody as pharmaceutical spiked into the rodent serum with varying concentrations and internal standard IgG with constant concentration. (B) Example MS spectrum with internal standard, biopharmaceutical and biopharmaceutical with extension at the N-terminus. (C) Example PK profiles of biopharmaceutical and biopharmaceutical with extension at the N-terminus in relative concentration units derived from comparing obtained rations to the calibration curve.

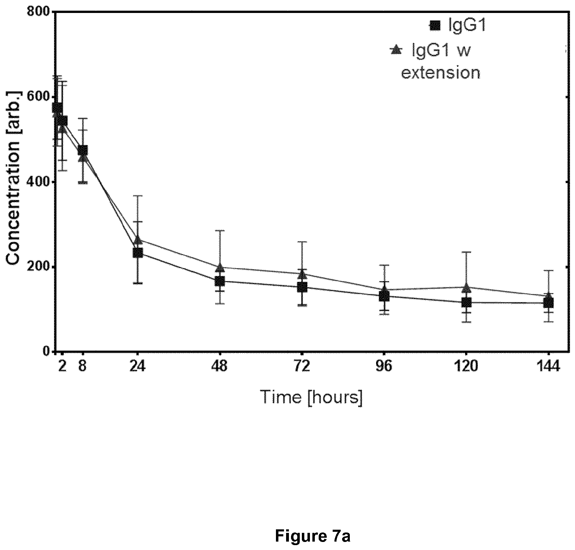

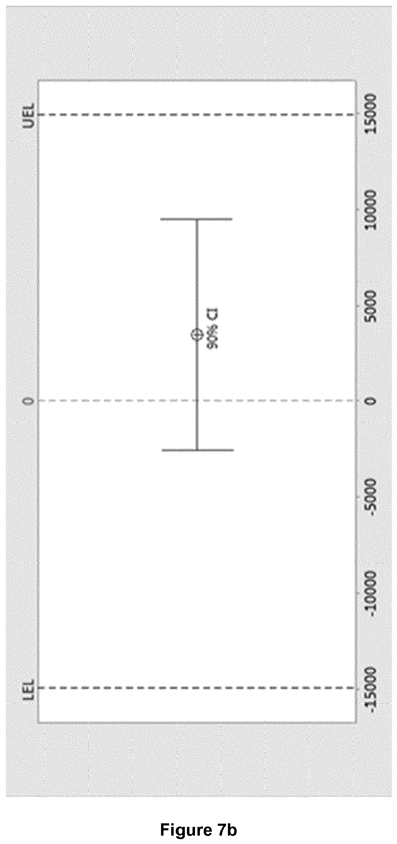

**Figure 7:** (A) PK profiles of an IgG1 biopharmaceutical (filled squares) and the leader peptide containing variant (filled triangle). (B) Equivalence test comparing AUCs of the leader peptide containing peptide variant and the non-leader peptide variant PK profiles.

## Detailed Description of the Invention

For the purposes of the present invention, the term "protein" refers to peptides and proteins, including antibodies and fusion proteins (e.g., Fc-fusion proteins).

The term "recombinant protein of interest" as used herein refers to a protein resulting from the expression of recombinant DNA within a living cell and may also be referred to as "recombinant protein". Recombinant DNA molecules are DNA molecules formed by laboratory methods of genetic recombination, such as molecular cloning, to bring together genetic material from multiple sources, creating sequences that would not otherwise be found in biological organisms. In the present context, the recombinant protein of interest also includes that the protein is not endogenous to the mammal of which the liquid sample was obtained. In other words, the recombinant protein has been administered to the mammal prior to obtaining the sample. Preferred recombinant proteins of interest are therapeutic recombinant proteins that have been typically been expressed in higher eukaryotic cells. In this regard, exemplary useful higher eukaryotic cells are selected from the following cell lines:

**Table 1**

| Cell Line | Meaning | Origin | Tissue Origin | Morphology |
|---|---|---|---|---|
| CHO | Chinese hamster ovary | Hamster | Ovary | Epithelium |
| COS-7 | *Cercopithecus aethiops,* origin-defective SV-40 | Ape - *Cercopithecus aethiops (Chlorocebus)* | Kidney | Fibroblast |
| BHK-21 | Baby hamster kidney fibroblast cells | Hamster | Kidney | Fibroblast |
| HEK-293 | Human embryonic kidney | Human | Kidney (embryonic) | Epithelium |
| HeLa | "Henrietta Lacks" | Human | Cervical cancer | Epithelium |
| HL-60 | Human leukemia | Human | Myeloblast | Blood cells |
| HUVEC | Human umbilical vein endothelial cell | Human | Umbilical vein endothelium | Epithelial |
| Jurkat | | Human | T cell leukemia | white blood cells |
| MCF-7 | Michigan Cancer Foundation-7 | Human | Mammary gland | Invasive breast ductal carcinoma |

(continued)

| Cell Line | Meaning | Origin | Tissue Origin | Morphology |
|---|---|---|---|---|
| NIH-3T3 | NIH, 3-day transfer, inoculum 3 x 10$^5$ cells | Mouse | Embryo | Fibroblast |
| RenCa | Renal carcinoma | Mouse | | Renal carcinoma |
| U937 | | Human | Leukaemic monocytic lymphoma | |
| Vero cells | *Vero* (truth) | African green monkey | Kidney epithelium | |

Particularly preferred in this regard are CHO cells or CHO derived cells (e.g., CHO-DXB11 or, CHO-DG44, which are widely used in the art to express biopharmaceutically useful recombinant proteins, such as antibodies.

The recombinant proteins of interest may be homologous to the host cell, or may be heterologous, i.e., foreign, to the host cell being utilized, such as, for example, a human protein produced by a Chinese hamster ovary (CHO) host cell. In certain embodiments, the proteins expressed by a host cell are directly secreted into the medium.

Examples of suitable mammalian, and in particular human, proteins include the following molecules: a cytokine; a cytokine receptor; a chemokine, such as TNF and TECK; a chemokine receptor, such as a TNFR and CCR9; a growth hormone, such as human growth hormone and bovine growth hormone; growth hormone releasing factor; parathyroid hormone; thyroid stimulating hormone; a lipoprotein; alpha-1-antitrypsin; insulin A-chain; insulin B-chain; proinsulin; follicle stimulating hormone; calcitonin; luteinizing hormone; glucagon; human macrophage inflammatory protein (MIP-1-alpha); a serum albumin, such as human serum albumin; mullerian-inhibiting substance; relaxin A-chain; relaxin B-chain; prorelaxin; mouse gonadotropin-associated peptide; a clotting factor, such as factor VIIIC, factor IX, tissue factor, and von Willebrand factor; an anti-clotting factor, such as Protein C; atrial natriuretic factor; lung surfactant; a plasminogen activator, such as urokinase or human urine or tissue-type plasminogen activator (t-PA); bombesin; thrombin; hemopoietic growth factor; enkephalinase; RANTES; a microbial protein, such as beta-lactamase; DNase; inhibin; activin; vascular endothelial growth factor (VEGF); a receptor for hormones or growth factors; an integrin; protein A or D; a rheumatoid factor; a neurotrophic factor such as bone-derived neurotrophic factor (BDNF), neurotrophin-3, -4, -5, or -6 (NT-3, NT-4, NT-5, or NT-6); a nerve growth factor, such as NGF-beta; platelet-derived growth factor (PDGF); a fibroblast growth factor, such as aFGF and bFGF; epidermal growth factor (EGF); a transforming growth factor (TGF) such as TGF-alpha and TGF-beta, including TGF-beta1, TGF-beta2, TGF-beta3, TGF-beta4, or TGF-beta5; insulin-like growth factor-I and -II (IGF-I and IGF-II); des(I-3)-IGF-I (brain IGF-I); an insulin-like growth factor binding protein, a CD protein, such as CD-3, CD-4, CD-8, and CD-19; erythropoietin; an osteoinductive factor, an immunotoxin; a bone morphogenetic protein (BMP); an interferon, such as interferon-alpha, -beta, and -gamma; a colony stimulating factor (CSF), such as M-CSF, GM-CSF, and G-CSF; an interleukin (ILs), such as IL-1 to IL-21; superoxide dismutase; a T-cell receptor; a cell surface membrane protein; a transport protein; a homing receptor; a regulatory protein; a decay accelerating factor; and a viral antigen, such as, a portion of the HIV-1 or HIV-2 envelope protein.

A preferred recombinant protein of interest in the context of the present invention is a fusion protein. The term "fusion protein" as used herein refers to a chimeric protein containing two proteins or protein fragments fused to each other (i.e., expressed as one polypeptide) often separated by an amino acid linker, preferably an effector domain fused to an Fc domain, albumin or transferrin. Thus, the fusion protein is preferably an Fc-fusion protein, a transferrin-fusion protein or an albumin-fusion protein, more preferably, the fusion protein is an Fc-fusion protein. Typically the Fc-domain in a fusion protein contains the CH2 and CH3 region and the hinge region of the IgG heavy chain, preferably of the IgG1 heavy chain. Fusion to an Fc-domain, albumin or transferrin usually increases the *in vivo* half-life and/or increases solubility of the effector domain. The amino acid linker may further contain a cleavage site for an endoproteinase. The effector domain may be a full length therapeutically relevant protein or a fragment thereof, particularly the extracellular part of a therapeutically relevant receptor or a membrane protein. Non limiting examples of therapeutically relevant Fc-fusion proteins are LFA-3/Fc (Alefecept), TNFR-Fc (Etanercept), CTLA-4/Fc (Abatacept and Belatacept), VEGFR1/2-Fc (Aflibercept), rFVIIIFc and rFIXFc, IL-1R1-Fc (Rilonacept), thrombopoietin-Fc (Romiplostim) and angiopoietin-1/2 antagonist peptide-Fc (Trebananib).

Examples of suitable mammalian, and in particular human, effector proteins suitable to be used in fusion proteins include the following molecules: a cytokine; a cytokine receptor; a chemokine, such as TNF and TECK; a chemokine receptor, such as a TNFR and CCR9; a growth hormone, such as human growth hormone and bovine growth hormone; growth hormone releasing factor; parathyroid hormone; thyroid stimulating hormone; a lipoprotein; alpha-1-antitrypsin; insulin A-chain; insulin B-chain; proinsulin; follicle stimulating hormone; calcitonin; luteinizing hormone; glucagon; human macrophage inflammatory protein (MIP-1-alpha); mullerian-inhibiting substance; relaxin A-chain; relaxin B-chain; prorelaxin; mouse gonadotropin-associated peptide; a clotting factor, such as factor VIIIC, factor IX, tissue factor, and von Willebrand factor; an anti-clotting factor, such as Protein C; atrial natriuretic factor; lung surfactant; a plasminogen activator, such as urokinase or human urine or tissue-type plasminogen activator (t-PA); bombesin; thrombin; hemopoietic growth factor; enkephalinase; RANTES; RANKL; a microbial protein, such as beta-lactamase; DNase; inhibin; activin; vascular endothelial growth factor (VEGF); a receptor for hormones or growth factors; an integrin; protein A or D; a rheumatoid factor; a neurotrophic factor such as bone-derived neurotrophic factor (BDNF), neurotrophin-3, -4, -5, or -6 (NT-3, NT-4, NT-5, or NT-6); a nerve growth factor, such as NGF-beta; platelet-derived growth factor (PDGF); a fibroblast growth factor, such as aFGF and bFGF; epidermal growth factor (EGF); a transforming growth factor (TGF) such as TGF-alpha and TGF-beta, including TGF-beta1, TGF-beta2, TGF-beta3, TGF-beta4, or TGF-beta5; insulin-like growth factor-I and -II (IGF-I and IGF-II); des(I-3)-IGF-I (brain IGF-I); an insulin-like growth factor binding protein, a CD protein, such as CD-3, CD-4, CD-8, and CD-19; erythropoietin; an osteoinductive factor, an immunotoxin; a bone morphogenetic protein (BMP); an interferon, such as interferon-alpha, -beta, and -gamma; a colony stimulating factor (CSF), such as M-CSF, GM-CSF, and G-CSF; an interleukin (ILs), such as IL-1 to IL-21; superoxide dismutase; a T-cell receptor; a cell surface membrane protein; a transport protein; a homing receptor; a regulatory protein; a decay accelerating factor; and a viral antigen, such as, a portion of the HIV-1 or HIV-2 envelope protein.

Another preferred recombinant protein of interest in the context of the present invention is an antibody. The term "antibody" as referred to herein includes whole antibodies and any antigen binding fragment (i.e., antigen-binding portion) or single chain thereof. It includes a polyclonal, monoclonal, bi-specific, multi-specific, human, humanized, or chimeric antibody. An "antibody" typically refers to a glycoprotein comprising at least two heavy (H) chains and two light (L) chains inter-connected by disulfide bonds, or an antigen binding portion thereof. Each heavy chain is comprised of a heavy chain variable region (abbreviated herein as $V_H$) and a heavy chain constant region. The heavy chain constant region is comprised of three domains, CH1, CH2 and CH3. Each light chain is comprised of a light chain variable region (abbreviated herein as $V_L$) and a light chain constant region. The light chain constant region is comprised of one domain, $C_L$. The $V_H$ and $V_L$ regions can be further subdivided into regions of hypervariability, termed complementarity determining regions (CDR), interspersed with regions that are more conserved, termed framework regions (FR). Each $V_H$ and $V_L$ is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system. Typically antibodies are glycosylated at the CH2 domain at asparagine residue N297. A significant number of antibodies also possess additional glycosylation sites (i.e., the Asn-X-Ser/Thr tripeptide) in their variable regions and N-linked glycosylation can be found in variable (V) domains of both heavy (VH) and light (VL) chains of serum IgG and of some monoclonal antibodies (mAbs). Examples of antigen-binding fragments encompassed within the term "antigen-binding portion" of an antibody include (i) a Fab fragment, a monovalent fragment consisting of the $V_L$, $V_H$, CL and CH1 domains; (ii) a F(ab')2 fragment, a bivalent fragment comprising two Fab fragments linked by a disulfide bridge at the hinge region; (iii) an Fd fragment consisting of the $V_H$ and CH1 domains; (iv) an Fv fragment consisting of the $V_L$ and $V_H$ domains of a single arm of an antibody, (v) a dAb fragment (Ward et al., Nature. 341:544-546 (1989)), which consists of a $V_H$ domain; and (vi) an isolated complementarity determining region (CDR) or (vii) a combination of two or more isolated CDRs which may optionally be joined by a synthetic linker. Furthermore, although the two domains of the Fv fragment, $V_L$ and $V_H$, are coded for by separate genes, they can be joined, using recombinant methods, by a synthetic linker that enables them to be made as a single protein chain in which the $V_L$ and $V_H$ regions pair to form monovalent molecules (known as single chain Fv (scFv); see e.g., Bird et al. Science 1988, 242:423-426; and Huston et al., Proc. Natl. Acad. Sci. USA 1988, 85:5879-5883). Such single chain antibodies are also intended to be encompassed within the terms antigen-binding portion and antigen-binding fragment of an antibody. These antibody fragments are obtained using conventional techniques known to those with skill in the art, and the fragments are screened for utility in the same manner as are intact antibodies.

The term "monoclonal antibody" (mAb), as used herein, refers to an antibody which displays a single binding specificity and affinity for a particular epitope. Accordingly, the term "human monoclonal antibody" refers to an antibody which displays a single binding specificity and which has variable and constant regions derived from human germ line immunoglobulin sequences. In one embodiment, human monoclonal antibodies are produced by a hybridoma which includes a B cell obtained from a transgenic non-human animal, e.g., a transgenic mouse, having a genome comprising a human heavy chain transgene and a light chain transgene fused to an immortalized cell.

The term "recombinant human antibody", as used herein, includes all human antibodies that are prepared, expressed, created or isolated by recombinant means, such as (a) antibodies isolated from an animal (e.g., a mouse) that is transgenic or transchromosomal for human immunoglobulin genes or a hybridoma prepared therefrom, (b) antibodies isolated from a host cell transformed to express the antibody, e.g., from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies have variable and constant regions derived from human germline immunoglobulin sequences. In certain embodiments, however, such recombinant human antibodies can be subjected to *in vitro* mutagenesis (or, when an animal transgenic for human Ig sequences is used, *in vivo* somatic mutagenesis) and thus the amino acid sequences of the $V_H$ and $V_L$ regions of the recombinant antibodies are sequences that, while derived from and related to human germline $V_H$ and $V_L$ sequences, may not naturally exist within the human antibody germline repertoire *in vivo.*

The term "humanized antibodies" according to the present invention refers to specific chimeric antibodies, immunoglobulin chains or fragments thereof (such as Fv, Fab, Fab', F(ab)2 or other antigen-binding subsequences of antibodies), which contain minimal sequence derived from non-human immunoglobulin. Preferably they contain or are modified to contain at least the portion of the CH2 domain of the heavy chain immunoglobulin constant region comprising the N-linked glycosylation site. For the most part, humanized antibodies are human immunoglobulins (recipient antibody) in which residues from a complementary -determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by the corresponding non-human residues. Furthermore, humanized antibodies can comprise residues which are found neither in the recipient antibody nor in the imported CDR or framework sequences. These modifications are made to further refine and maximize antibody performance. In general, the humanized antibody will comprise at least one, and typically two, variable domains, in which all or substantially all off the CDR regions correspond to those of a non-human immunoglobulin and all or substantially all of the framework regions are those of a human immunoglobulin consensus sequence. Preferably, the humanized antibody also comprises at least a portion of an immunoglobulin constant region, typically that of a human immunoglobulin.

The term "endogenous antibody" as used herein refers to antibodies endogenous to the mammal the sample to be analyzed is derived from, i.e., produced by said mammal, whereas the recombinant protein of interest to be purified and analyzed from that sample is heterologous to the mammal of which the sample to be analyzed is derived from.

The term "peptide" as used herein refers to chains of amino acid monomers linked by peptide amide bonds. Peptides may be short chains of about 10 amino acids or less. Peptides may also be long chains of about 70 amino acids or more or anything in between. In the context of the present invention the fragments of a recombinant protein of interest released by an endoproteinase is referred to as a peptide or glycopeptide, if it carries a glycan.

The term "protein variant" as used herein refers to any modification of a protein resulting in different variants or isoforms of a protein. Without being limited thereto, examples are modifications or variants due to deamidation, oxidation, N-terminal heterogeneity, C-terminal heterogeneity, isomerization (isoaspartate), glycation or disulfide isoforms. Protein variants include modifications due to chemical degradation pathways, such as deamidation and oxidation, but also incomplete processing during protein biosynthesis, such as a remaining N-terminal leader peptide. Some variations, such as deamidation, glycation and C-terminal lysine cleavage, leads to a decrease in pl value and the formation of acidic variants. Basic variants can result from the presence of C-terminal lysine or glycine amidation, succinimide formation or amino acid oxidation. The protein variants to be analyzed using the methods of the invention do not include glycosylation or glycosylation variants. The protein variants have in common that they can be analyzed as protein or peptide variations, while analysis of glycosylation variants usually requires chemical or enzymatic release of the oligosaccharides prior to analysis.

The term "glycosylation variants" as used herein refers to any modification of a glycan, particularly glycans in N- or O-glycosylated proteins. For the purposes of the present invention, a "glycosylation" or a "glycan" refers to a sugar or an assembly of sugars (*i.e.,* saccharide or carbohydrate), typically enzymatically attached to another molecule, such as proteins or lipids, but also in its free form. N-glycosylation analysis is different because of countless N-glycan variants which may be attached to the protein molecules and the huge differences in their relative amounts. Analysis of glycosylation variants therefore differs from analysis of protein variants regarding the analytics applied and the reference standard used and is described in details in EP 2 975 401. Glycosylation is to be distinguished from glycation.

For the purposes of the present invention, a "glycan" refers to any sugar or assembly of sugars (*i.e.,* saccharide or carbohydrate), typically enzymatically attached to another molecule, such as proteins or lipids, but also in its free form. As referred to herein, an "N-glycan" is a glycan covalently linked to an asparagine residue of a polypeptide chain in the consensus sequence: -Asn-X-Ser/Thr (*e.g.,* comprising the common core structure Man$\alpha$1-6(Man$\alpha$1-3)Man$\beta$1-4GlcNAc$\beta$1-4GlcNAc$\beta$1-N-Asn). As referred to herein, an "acidic glycan" is an N-glycan containing at least one terminal sialic acid (at the non-reducing terminus). As referred to herein, a "neutral glycan" is an N-glycan that does not contain any sialic acid. As referred to herein, "glycosylation" is the enzyme-catalyzed covalent attachment of a carbohydrate to a polypeptide, lipid, polynucleotide, carbohydrate, or other organic compound, generally catalyzed by glycosyltransferases, utilizing specific sugar nucleotide donor substrates. For the purposes of the present invention, a "polysaccharide" is a glycan composed of repeating monosaccharides, preferably greater than ten monosaccharide units in length. As referred to herein, a "sugar" refers to any carbohydrate, preferably to low molecular weight carbohydrates that are sweet in taste (see glossary of Essentials of Glycobiology. 2nd edition. Varki A, Cummings RD, Esko JD, et al., editors. Cold Spring Harbor (NY): Cold Spring Harbor Laboratory Press; 2009). The following abbreviations for monosaccharides are used: N-acetylglucosamine (GlcNAc), fucose (Fuc), mannose (Man), galactose (Gal), and sialic acid (SA). Carbohydrate moieties are described herein with reference to commonly used nomenclature for oligosaccharides. A review of carbohydrate chemistry which uses this nomenclature can be found, for example, in Hubbard and Ivatt, Ann. Rev. Biochem. 50, 555-583 (1981).

The term "deamidation" as used herein refers to a chemical reaction in which an amide functional group is removed from the amino acids asparagine and glutamine in a protein. This is an important mechanism in the degradation of proteins, because it damages the amide-containing side chains of the amino acids asparagine and glutamine. Deamidation of asparagine results in either aspartate or isoasparate. This process is considered a deamidation because the amide in the asparagine side chain is replaced by a carboxylate group. Deamidation of glutamine results in the two isomeric products $\alpha$- and $\gamma$-glutamic acid. A similar reaction can also occur in aspartate side chains yielding a partial conversion to isoaspartate, referred to herein by "isoformation". Formation of isoasparate from aspartate is also a protein variant according to the invention. Deamidation is a process that may impair functioning and/or pK of the recombinant protein of interest. Deamidation can be analyzed on the peptide level after enzymatic digestion by e.g. HPLC, CE, HPLC-MS or CE-MS methods.

The term "oxidation" as used herein refers to a molecular modification in proteins by an oxidizing agent or oxidizing stress, such as reactive oxygen or nitrogen species. Oxidation may lead to fragmentation of polypeptide chains, oxidation of amino acid side groups and protein cross-linking. Of the 20 proteinogenic amino acids more than half can be modified by oxidative stress resulting, e.g., in reactive aldehyde groups that can irreversibly react with amino groups of lysine or the N-terminus to form a Schiff's base. Further oxidation of thiol groups may lead to disulfide bonds and two tyrosine molecules can form a dityrosine. Particularly prone to oxidation are the amino acids methionine, cysteine, lysine, histidine and tryptophane. Oxidation may affect critical amino acids involved in antigen binding and may therefore impair functioning and/or pK of the recombinant protein of interest. Oxidation can be analyzed on the intact, reduced or peptide level after enzymatic digestion by e.g. HPLC, CE, HPLC-MS or CE-MS methods.

The term "N-terminal heterogeneity" as used herein refers to any modification at the N-terminus of a recombinant protein of interest, including containing to some extend a N-terminal leader peptide extension. Antibodies can contain variations at their N-termini of both heavy and light chain. These extensions can result from incomplete processing during protein biosynthesis. The extension can for example contain charged or hydrophobic amino acids that can influence the physico-chemical properties of the antibody, such as antigen binding. N-terminal heterogeneity is a process that may impair functioning and/or pK of the recombinant protein of interest. N-terminal heterogeneity can be analyzed on the intact, reduced or peptide level after enzymatic digestion by e.g. HPLC, CE, HPLC-MS or CE-MS methods.

The term "C-terminal heterogeneity" as use herein refers to any modification at the C-terminus of a recombinant protein of interest, such as C-terminal lysine cleavage and C-terminal lysine and glycine amidation ($NH_2$ formation or loss of COOH). C-terminal heterogeneity is a process that may impair functioning and/or pK of the recombinant protein of interest. C-terminal heterogeneity can be analyzed on the intact, reduced or peptide level after enzymatic digestion by e.g. HPLC, CE, HPLC-MS or CE-MS methods.

The term "glycation" as used herein refers to a non-enzymatic attachment of a monosaccharides such as fructose, mannose or glucose to N-terminal amino groups or lysine side chains of a protein, i.e., without the controlling action of an enzyme. It is to be distinguished from "glycosylation". Glycation is a process that may impair functioning and/or pK of the recombinant protein of interest. Glycation can be analyzed on the intact, reduced or peptide level after enzymatic digestion by e.g. HPLC, CE, HPLC-MS or CE-MS methods.

The term "disulphide isoforms" as used herein refers to disulfide bonds within the IgG2 antibody class that can form more than one pair combination, i.e., alternative linkages. IgG2 antibody heavy chains are connected by four interchain disulfide bridges. Disulfide shuffling/rearrangement leads to various structural disulfide isoforms. The main isoforms are named IgG2-A; IgG2-A/B and IgG2-B (Dillon et al. JBC 2008, 283(23), 16206-16215, Liu et al JBC 2008, 283(43), 29266-29272; Figure 1). These isoforms have a different three dimensional structure which can lead to differences in the structure, function or PK. Since disulfide isoforms do not differ in their mass, they are typically analyzed using capillary electrophoresis or HPLC.

As used herein "affinity ligand" refers to a ligand that binds specifically to the recombinant protein of interest. This affinity ligand is used to separate the recombinant protein of interest from the other compounds in the mammalian liquid sample, such as serum or plasma, using affinity chromatography. Typically, if the recombinant protein of interest is an antibody, the affinity ligand is an antigen. For other recombinant proteins, the affinity ligand is typically a binding partner or a substrate of said recombinant protein. If the recombinant protein of interest is a fusion protein the affinity ligand is typically a binding partner or a substrate of the effector domain of the fusion protein, e.g., the ligand for a receptor or *vice* versa. Alternatively the affinity ligand may be an antibody specific for said recombinant protein of interest or an antibody specific for said effector domain of the fusion protein. Preferably, the affinity ligand binds to the recombinant protein of interest in the nM range, more preferably in the pM range. The skilled person will understand that Protein A and Protein G or any other generically Fc-domain binding protein is not suitable as an affinity ligand specific for a recombinant Fc-fusion protein or antibody of interest in a liquid samples of a mammal containing endogenous antibodies, particularly in serum and plasma.

In case the recombinant protein of interest is an antibody and it is purified from the sample via binding of the variable region of the antibody to the affinity ligand immobilized on the solid support, the protein variation should not substantially affect antibody binding. Not substantially affecting antibody binding means that the binding affinity of the protein variants to the affinity ligand should not differ by more than 40%, more than 30%, more than 20%, more than 10% or more than 5%, when determined, e.g., by surface plasmon resonance (SPR) technology such as in a BIACORE 3000 instrument. Preferably the affinity ligand is an antigen.

In case the recombinant protein of interest is a fusion protein, such as a Fc-fusion protein, and it is purified from the sample via binding of the effector domain of the fusion protein to the affinity ligand immobilized on the solid support, the protein variation should not substantially affect fusion protein binding. Not substantially affecting fusion protein binding binding means that the binding affinity of the protein variants to the affinity ligand should not differ by more than 40%, more than 30%, more than 20%, more than 10% or more than 5%, when determined, e.g., by surface plasmon resonance (SPR) technology such as in a BIACORE 3000 instrument. Preferably the affinity ligand is a binding partner or an antibody specific to the effector domain.

The term "antigen" as uses herein is a substance which provokes an adaptive immune response and may also be referred to as immunogen. An antigen binds to the antigen-binding site of an antibody. Antigens are typically of high molecular weight and proteins or polysaccharides. As used herein an antigen may also refer to the immunogenic part of an antigen comprising the epitope, e.g., peptides. Peptides, lipids, nucleic acids and many other materials can also function as antigens. Immune responses may also be generated against smaller substances, called haptens, if these are chemically coupled to a larger carrier protein, such as bovine serum albumin, keyhole limpet hemocyanin (KLH) or other synthetic matrices.

As used herein, "specific binding", "selective binding" and "selectively binds" and "ligand specific for", refer to an affinity ligand, binding to a predetermined recombinant protein of interest, such as the effector domain of a fusion protein or to an antibody. For example, in one embodiment, the ligand binds with an affinity (KD) of approximately less than $10^{-7}$ M, such as approximately less than $10^{-8}$ M, $10^{-9}$ M or $10^{-10}$ M, $10^{-11}$ M or even lower to the recombinant protein when determined, e.g., by surface plasmon resonance (SPR) technology such as in a BIACORE 3000 instrument, and binds to the predetermined recombinant protein of interest and the internal standard with an affinity that is at least two-fold greater than its affinity for binding to a non-specific antigen or other proteins in the liquid sample. Preferably, the affinity ligand binds to the recombinant protein of interest in the nM range, more preferably in the pM range. Thus, in the context of the invention "a ligand specific for" means that the ligand preferentially binds to the recombinant protein of interest and thus separates said recombinant protein of interest from other proteins present in the sample, particularly from other antibodies present in the sample. The phrases "an antibody recognizing an antigen" and "an antibody specific for an antigen" are used interchangeably herein with the term "an antibody which selectively binds to an antigen".

The term "eluate" as used herein relates to the material released from the solid support. It may comprise the buffer or solution used during elution or a different buffer or solution.

The term "elution buffer" as used herein relates to a buffer or solution used to release the recombinant protein of interest and the internal standard from the affinity ligand. Typically the buffer is of low pH, preferably a pH of < 4, more preferably a pH of < 3.5 and even more preferably a pH of < 3. A suitable buffer would be, e.g., a glycine buffer of pH < 4, such as a glycine buffer of about pH 2.7. The buffer may further contain one or more chaotropic reagent, such as guanidinium chloride, butanol, ethanol, propanol, lithium perchlorate, lithium acetate, urea, thiourea or magnesium chloride. Preferred examples of a chaotropic reagent are guanidinium chloride or urea. Guanidinium chloride may be used at 2 to 6 M, preferably at about 4-6 mM, more preferably at 6 mM and urea may be used at 1-8 M, preferably at about 4-6 mM, more preferably at 4 mM. Thus, the elution buffer may contain guanidinium chloride at 2 to 6 M or urea at 1-8 M and more preferably guanidinium chloride at about 4-6 M or urea at about 4-6 M and even more preferably guanidinium chloride at about 6 mM and urea at about 4 mM.

As used herein "analytical separation method" relates to chromatographic analytical methods but also includes capillary electrophoresis. Thus, any analytical method suitable to distinguish the respective protein variant would be suitable in the context of the present invention. It includes HPLC, CE and MS. Wherein MS may also be combined with HPLC or CE in a single analytical approach.

As used herein, "LC" means liquid chromatography and is a separation technique in which the mobile ühase is a liquid. Present day LC that generally utilizes very small packing particles and a relatively high pressure is referred to as high performance liquid chromatography (HPLC). LC and HPLC are used interchangeably herein. LC may be combined with mass spectrometry, referred to as "LC-MS" herein. LC-MS may further include tandem MS. The term "reversed phase LC" or "reverse phase HPLC" (RP-LC or RP-HPLC) as used herein has a non-polar stationary phase and an aqueous, moderately polar mobile phase, *e.g.,* a silica which has been surface-modified with $RMe_2SiCl$, where R is a straight chain alkyl group such as $C_{18}H_{37}$ or $C_8H_{17}$ (Lehto and Hou, Chemistry and Analysis of Radionuclides, Wiley-VCH Verlag & Co., Weinheim, Germany, 2011, page 170). In the context of the present invention "reverse phase LC-MS" is preferred. The mobile phase used in reverse phase is more compatible with MS and therefore more sensitive than normal phase LC-MS.

As used herein "nano-LC" or nano-HPLC (RP-nano-LC or RP-nano-HPLC) is characterized by a decreased inner diameter of the columns that are used for LC (10-150 μm) and smaller flow-rates (10-1000 nl/min) compared to conventional LC or HPLC, respectively. This down-scaling results in high plate counts of the nano-LC system and the ability to analyze proteinaceous samples in the low femtomole and subfemtomole ranges (Chervet et al., Analytical Chemistry 1996, 68:1507-12). Consistent with the understanding and common general knowledge in the field of liquid chromatography, it will be appreciated that in accordance with the invention nano-LC and nano-HPLC are suitable and intended forms of LC and HPLC, respectively, for the purposes of the present invention; and that RP-nano-LC and RP-nano-HPLC are suitable and intended forms and even preferred forms of RP-LC and RP-HPLC, respectively. The same applies to the likewise well-known and established techniques of *micro-LC* and *capillary-LC*, or *RP-micro-LC* and *RP-capillary-LC*, which for the purposes of the present invention are suitable and intended forms of LC, and RP-LC, respectively. Where the terms LC, HPLC, LC-MS or HPLC-MS is used herein, this also encompasses their preferred embodiments, nano-LC, nano-HPLC, nano-LC-MS or nano-HPLC-MS and their reverse phase forms.

For the purposes of the present invention, a "mobile phase" of RP-LC or RP-HPLC is preferably a gradient of an organic modifier (*e.g.,* acetonitrile or methanol) in water, with an ionic modifier that controls the pH and ionization state or acts as an ion pairing reagent. Anionic ion-pair reagents (e.g., trifluoroacetic acid (TFA)) bind to protonated basic groups of peptides. The addition of 0.1% TFA acidifies the eluent which causes the carboxylic groups of peptides and proteins to become protonated, resulting in a larger hydrophobicity of the molecules. Cationic ion-pairing reagents (*e.g.,* triethylammonium ions) bind to ionized carboxyl groups of peptides ("Protein Liquid Chromatography", Journal of Chromatography Library, vol. 61, edited by Kastner M, Elsevier Science B.V., 2000, page 153). Diethylamine (DEA) can also be used as an ion pairing reagent (Melmer et al., Journal of Chromatography A (2011), Volume: 1218(1): 118-123). For normal phase or HILIC chromatography a suitable mobile phase consists for example of 60 mMol ammonium formate in 75% acetonitrile (mobile phase A) and 115 mMol ammonium formate in 54% acetonitrile (mobile phase B) (Melmer et al., Anal Bioanal Chem (2010), Volume 398: 905-914).

As used herein, "ion trap mass spectrometry" is an arrangement in which ions with a desired range of quotients mass/charge are first made to describe stable paths under the effect of a high-frequency electric quadrupole field, and are then separated and presented to a detector by adjusting the field so as to selectively induce path instability according to their respective mass/charge ratios *e.g.,* quadrupole ion trap (see http://www.genomicglossaries.com/content/mass_spectrometry.asp). Sensitivity of the methods of the invention can be improved by using more sensitive nano ESI sources.

For the purposes of the present invention, all "N-glycans" are understood to have the common core sugar sequence, Manα1-6(Manα1-3)Manβ1-4GlcNAcβ1-4GlcNAcβ1-Asn-X-Ser/Thr, and are classified into three types: (1) "oligomannose" (high mannose), in which only mannose residues are attached to the core; (2) "complex", in which "antennae" initiated by N-acetylglucosaminyltransferases (GlcNAcTs) are attached to the core; and (3) "hybrid", in which only mannose residues are attached to the Manα1-6 arm of the core and one or two antennae initiated by N-acetylglucosaminyltransferases (GlcNAcTs) are on the Manα1-3 arm (Essentials of Glycobiology, 2nd edition, Varki et al., editors, Cold Spring Harbor (NY): Cold Spring Harbor Laboratory Press; 2009; Chapter 8).

For the purposes of the present invention, a reference to the analysis of the protein variants or fragments thereof, "in one run", "in one approach" or "in a single (analytical) approach" means that the MS analysis is directly coupled to (or in other words, is performed on-line with) the LC or RP-LC step. This means that there is no further analytical or preparatory step between the RP-LC and the MS analysis. It will be understood that this does not exclude that fluorescence detection will occur between the RP-LC and the MS. This is also referred to as "LC-MS" or RP-LC-MS".

The term "enzymatic cleavage" as used herein refers to any cleavage of a protein that involves an enzyme and allows for release of a fragment of the recombinant protein while avoiding elution of most non-specifically bound proteins. The enzymatic cleavage may be done using an endoproteinase. The enzyme should be selective for the recombinant protein of interest. This means that the specific cleavage site is rare and preferably only present in the recombinant protein of interest or additionally in a few other proteins, such as a specific family of proteins, or a group of proteins all comprising the same domain. A more selective enzyme avoids elution of fragments of non-specifically bound proteins from the solid support. Preferably the enzyme cleaves the recombinant protein of interest efficiently and specifically (e.g., only at a known cleavage site). Suitable enzymes are for example endoproteinases that cleave within the recombinant protein of interest specifically at a certain consensus sequence and that have narrow substrate specificity (or high selectivity). Preferably, the endoproteinase is specific (and/or selective) for the recombinant protein of interest or a domain contained in the recombinant protein of interest. For example, a suitable endoproteinase specifically only cleaves the recombinant protein of interest or a group of proteins comprising essentially the same domain, including the recombinant protein of interest. Examples for a group of proteins comprising essentially the same domain are antibodies and Fc-fusion proteins, all containing an Fc-domain. A suitable enzyme has preferentially only one cleavage site in the recombinant protein of interest or within each identical polypeptide chain within the recombinant protein of interest. The terms "endopeptidase", endoproteinase", "proteinase" or "protease" are used interchangeably herein.

For example endoproteinases suitable in the methods of the invention to release the Fc-domain of the recombinant protein containing a Fc-domain from the solid support are endoproteinase, such as papain, ficin, cysteine protease SpeB (FabULOUS) or cysteine proteinase IdeS (FabRICATOR®), preferably the endoproteinase is the cysteine proteinase IdeS (FabRICATOR®). For example IdeS specifically cleaves human IgG in the hinge region between the two glycines of the constant sequence ELLGGPS and SpeB cleaves in the hinge region between threonine and cysteine within the sequence KTHTCPPC.

Alternatively, fusion proteins such as Factor IX-albumin (FIX-albumin) comprise a linker sequence between the two domains that is based on amino acids 137-153 derived from the N-terminus of the activation peptide of FIX. Activation of FIX-recombinant albumin fusion protein by either FXIa or FVIIa/TF cleaves the linker, thereby separating the FIXa and rHA moieties of the fusion protein. Thus, FXIa and/or FVIIa/TF are suitable to cleave FIX-albumin selectively. Also Factor Xa is a site-specific protease that exhibit very low non-specific cleavage under many conditions. Thus, Factor Xa may be used for cleaving proteins that contain a Factor Xa cleavage site (cleavage behind arginine of the sequence Ile-Glu/Asp-Gly-Arg). Factor Xa kits are e.g. available from Novagen (69036-3). Likewise thrombin is a site-specific protease for specific cleavage between arginine and glycine of the sequence LeuValProArgGlySer in recombinant fusion proteins.

The term "solid support" as used herein refers to any solid surface that can be used to immobilize a ligand thereon, particularly in affinity chromatography. Typically a "solid support" suitable for affinity chromatography is a resin, such as a resin comprising microbeads with a large surface area. Particularly suitable resins in the present invention are sepharose beads, agarose beads or magnetic beads, wherein magnetic beads are preferred. The term "immobilized" refers to covalent or non-covalent binding to the solid support, either directly or indirectly. Typically the affinity ligand is covalently coupled to the solid support, for example via N-hydroxysuccinimide (NHS), cyanogen bromide, epoxide, carboiimide or thiopropyl reactive groups. Commercially available microbeads activated with one of the above reactive groups are known in the art, e.g., NHS-activated sepharose. Once the recombinant protein binds to its specific affinity ligand immobilized or coupled to the solid support the recombinant protein is immobilized on the solid support via non-covalent binding to the affinity ligand.

The term "liquid sample of a mammal" as used herein refers to any liquid sample obtained from a mammal at a certain time point comprising biological material such as cells, protein, DNA or RNA. Typically the liquid sample is a body fluid, such as serum or plasma. However, the liquid sample may also be whole blood, urine, cerebral spinal fluid, amniotic fluid, saliva, sweat, ejaculate, tears, phlegm, vaginal secretion, vaginal wash or colonic wash. Preferably the sample is cleared from cells or debris, e.g., by centrifugation prior to be subject to the methods of the invention. For the liquid sample to contain the recombinant protein of interest, the sample must have been obtained following administration of said recombinant protein of interest to the mammal. In the context of the present invention the recombinant protein is to be understood as a biopharmaceutical or therapeutically active recombinant protein. The skilled person will understand that a liquid sample, or an "aliquot" of a liquid sample, may be used in the present invention. If only an aliquot of the liquid sample is used to be analyzed according to the methods of the present invention, other aliquots of the same sample may be analyzed for different parameters, such as protein concentration, or stored frozen for later analysis.

The term "lower limit of quantification (LLOQ)" as used herein refers to the lowest concentration of an analyte in a sample that can be quantitatively determined with suitable precision and accuracy", wherein the analyte is the recombinant protein. The LLOQ is typically given as $\mu$g/ml. the skilled person understands that it is therefore strongly dependent on the sample volume used for analysis.

The term "internal standard" as used herein refers to a protein which binds to the affinity ligand with an affinity comparable to the recombinant protein of interest and is distinguishable from the recombinant protein of interest using a suitable analytical separating method. Thus, when using MS for analysis, the internal standard should have a different mass, when using HPLC the internal standard should have a different retention time and when using capillary electrophoresis the internal standard should have a different electrophoretic mobility or effective net charge. The internal standard according to the invention is co-purified with the recombinant protein of interest and therefore typically spiked into the liquid sample of a mammal or an aliquot thereof prior to affinity purification. The skilled person will understand that the internal standard may be added at any time point prior to separation of the recombinant protein of interest from the sample. Since the internal standard is co-purified, it is important that it binds to the affinity ligand with comparable or substantially the same affinity as the recombinant protein of interest. This ensures that the internal standard is purified with the same efficacy as the recombinant protein of interest to control for variations during purification between different samples. The internal standard also allows for quantification of the presence of the individual protein variants within a sample, including absolute quantification. The skilled person would know what a substantially same affinity would be. Typically, the internal standard has a slightly lower binding affinity compared to the recombinant protein of interest, including a difference of less than 40%, less than 30%, less than 20%, less than 10% or less than 5%, when determined, e.g., by surface plasmon resonance (SPR) technology such as in a BIACORE 3000 instrument. It would also be apparent to a skilled person that a higher degree of similarity in binding affinity is preferable and allows for more reliable results, comparability of the samples and more accurate quantification. In case the recombinant protein of interest is an antibody purified via binding to its antigen, the internal standard may be, e.g., an antigen-binding fragment of the antibody or a Fc-fusion protein binding with similar affinity to the affinity ligand or *vice versa.* In case the recombinant protein of interest is an antibody purified via specific binding to its Fc portion, the internal standard may be, e.g., an antibody of the same subclass, but different antigen specificity or an Fc-fusion protein. In this case it must be ensured that the purification step is specific to the recombinant protein of interest and the internal standard and avoids co-purification of endogenous antibodies in the sample. For example in a pre-clinical study in rabbits, a human or humanised IgG antibody can be purified from a rabbit serum sample using an anti-human IgG antibody or even a subtype specific anti-human IgG antibody. The internal standard is further standardized, which means that the identical standard is added to each sample of the experiment or to each sample that is compared to each other. Preferably, the internal standard is stored frozen until use. It is also preferably that the standard is added to the two or more samples at a constant concentration. The use of an internal standard compensates variations in the sample preparation and allows for more reliable results using an analytical separation method, such as HPLC, CE or MS. It is therefore preferably added as early as possible to the sample. Moreover, the reference standard allows for analyzing the protein variants individually. This means the amount of each protein variant can be quantified individually relative to the respective internal standard, rather than the relative proportion of the variants within each sample. Hence, PK parameter of individual protein variants can be determined.

The term "high throughput" as used herein relates to a mode or method that permits rapid and highly parallel sample preparation of a number of samples, such as more then 10, more than 50, more than 100, more than 500 or more than 1000 samples.

For the purposes of the present invention, a reference to % as used in the examples will be understood to refer to (v/v) unless explicitly stated otherwise.

As used herein, the term "about" when used together with a numerical value (*e.g.,* a pH value or a percentage value) is intended to encompass a deviation of 20%, preferably 10%, more preferably 5%, even more preferably of 2%, and most preferably of 1% from that value. When used together with a numerical value it is at the same time to be understood as individually disclosing that exact numerical value as a preferred embodiment in accordance with the present invention.

As used herein, the term "comprising" is to be construed as encompassing both "including" and "consisting of", both meanings being specifically intended, and hence individually disclosed, embodiments in accordance with the present invention.

Accordingly, the present invention relates to a method of analyzing protein variants of a recombinant protein of interest in liquid samples of a mammal comprising a) providing two or more liquid samples of a mammal comprising the recombinant protein of interest; b) immobilizing the recombinant protein of each of said samples on a separate solid support coupled to an affinity ligand specific for the recombinant protein in the samples; c) eluting the recombinant protein or a fragment thereof of each of said samples from the solid support into separate eluates; and d) analyzing the protein variants of step c) of each of said samples separately using an analytical separating method and comparing said two or more samples, wherein an internal standard binding to the same affinity ligand is added to each of said samples prior to step b and the internal standard is analyzed together with the recombinant protein of step d), and wherein the protein variants of the recombinant protein of interest to be analyzed are due to deamidation, oxidation, N-terminal heterogeneity, C-terminal heterogeneity, isomerization, or disulfide isoforms.

More specifically the method of the present invention may be used for analyzing one or more pharmacokinetic parameter of said protein variants.

Thus, according to the methods of the invention, the internal standard is preferably added to the sample during or prior to immobilizing the recombinant protein of interest according to step b), preferably prior to immobilizing the recombinant protein of interest according to step b).

The analytical separating method according to the invention may be, without being limiting thereto, HPLC, capillary electrophoresis (CE) or mass spectrometry (MS). MS explicitly includes HPLC-MS and CE-MS. Other methods for analyzing protein variants are known in the art and a method is suitable in the context of the present invention if it is able to separate and hence distinguish the specific protein variants of the recombinant protein of interest.

The protein variants of the recombinant protein of interest to be analyzed using the methods of the invention may be due to deamidation, oxidation, N-terminal heterogeneity, C-terminal heterogeneity, isomerization, glycation or disulfide isoforms.

The skilled person would know that a suitable analytical separating method to be used in the methods of the invention depends on the specific protein variants to be analyzed. For example protein variants of the recombinant protein of interest due to deamidation, oxidation, N-terminal heterogeneity, C-terminal heterogeneity, isomerization or glycation may be analyzed using an analytical separating method selected from the group consisting of HPLC, CE and MS. Further, protein variants of the recombinant protein of interest due to disulfide isoforms may be analyzed using the analytical separating method selected from the group consisting of HPLC and CE. Since the disulfide isoforms do not differ in mass, MS is not suitable to distinguish the isoforms. The skilled person will understand that MS may be coupled to HPLC or CE.

Affinity purification from liquid samples of a mammal, such as a serum samples, may require a pre-clearing step. A per-clearing step is particularly advantageous, if contaminating endogenous proteins are present in the sample that would also bind to the affinity ligand. The pre-clearing step may comprise i) immobilizing the Fc-containing protein of interest on a solid support using an Fc-binding protein, wherein said Fc-binding protein is preferably selected from protein G or protein A, more preferably said Fc-binding protein is protein G; and ii) eluting the Fc-containing protein of interest; wherein the solid support used in said pre-clearing step is made of the same material as the solid support used for immobilizing the recombinant protein of interest (step b) without the coupled affinity ligand. Thus, the pre-clearing step may be an upstream second affinity purification step immobilizing the recombinant protein on the solid support followed by eluting the recombinant protein from the solid support.

According to the methods of the present invention the protein variants of the recombinant protein of interest do not include glycosylation variants, such as N-glycans or O-glycans. N-glycans may be of the high mannose type, hybrid type or complex type N-glycans.

The internal standard used in the methods according to the invention is a protein binding to the affinity ligand with an affinity comparable to the recombinant protein of interest that is distinguishable from the recombinant protein of interest using the analytical separating method.

The use of an internal standard compensates variations in the sample preparation and analysis, resulting in more precise results. It is therefore preferably added as early as possible to the sample. Moreover, the reference standard allows for analyzing the protein variants individually as the samples can be normalized. This means the amount of each protein structure can be quantified individually relative to the internal standard based on the known concentration of the internal standard added to the sample. Hence, PK parameter of individual protein variants can be determined. The internal standard may be the same recombinant protein as the recombinant protein of interest, additionally carrying a label or a tag that allows distinguishing it from the recombinant protein of interest. It may also be a similar protein such as a Fc-fusion protein in case the protein of interest was an antibody or an antibody in case the protein of interest was an Fc-fusion protein. It may also be a different antibody as long as it binds to the affinity ligand with substantially the same affinity. A different antibody may be an antibody of a different isotype, or binding to a different epitope on the affinity ligand. Alternatively a different antibody may be an antibody of the same isotype with a different binding specificity in case the Fc-domain of the antibody binds to the immobilized affinity ligand. The internal standard should be added to the sample at a known concentration. Preferably a calibration curve is calculated for the recombinant protein of interest comprising spiking the recombinant protein of interest into a liquid sample of a mammal at varying concentrations and spiking the internal standard into the same sample at a constant concentration, wherein the liquid sample is of the same type as the liquid sample of a mammal to be analyzed according to the methods of the inventions, e.g., serum or plasma. Using a reference standard as described above allows for determining the relative amount of each protein variation separately.

The recombinant protein analyzed in the methods of the invention is preferably a fusion protein or an antibody, more preferably an Fc-fusion protein or an antibody, more preferably an antibody. In step c of the methods of the invention the recombinant protein of interest or a fragment thereof may be eluted. In embodiments where the recombinant protein of interest is an Fc-fusion protein or antibody, the fragment of the recombinant protein of interest released from the solid support in step c) may be an Fc domain.

For Fc-domain containing recombinant proteins of interest, the method of the invention may comprises the following steps: a) providing two or more liquid samples of a mammal comprising the recombinant protein of interest having an Fc-domain; b) immobilizing the recombinant protein of each of said samples on a separate solid support coupled to an affinity ligand specific for the recombinant protein of interest in the samples; c) releasing a fragment of the recombinant protein of interest of each of said samples, preferably the Fc-domain, from the solid support into separate eluates by enzymatic cleavage of the recombinant protein of interest; and d) analyzing the protein variants of step c) of each of said samples separately using an analytical separating method and comparing said two or more samples, wherein an internal standard binding to the same affinity ligand is added to each of said samples prior to step b and the internal standard is analyzed together with the recombinant protein of interest of step d), and wherein the protein variants of the recombinant protein of interest to be analyzed are due to deamidation, oxidation, N-terminal heterogeneity, C-terminal heterogeneity, isomerization, or disulfide isoforms.

In one particularly preferred embodiment, the recombinant protein is an antibody and the variable region of the antibody binds to the affinity ligand immobilized on the solid support, preferably the affinity ligand is an antigen. This is particularly suitable when the protein variation does not affect antibody binding. In a preferred embodiment the antibody is an IgG antibody selected from IgG1, IgG2, IgG3 or IgG4, more preferably an IgG1 or IgG2 antibody. The antibody may be a chimeric, human or humanized IgG antibody, preferably the antibody is a human or humanized IgG1 or IgG2 antibody.

In another particularly preferred embodiment the recombinant protein is an Fc-fusion protein comprising an Fc-domain and an effector domain and the effector domain binds to the affinity ligand immobilized on the solid support, wherein the affinity ligand is a binding partner or an antibody specifically binding to the effector domain of the Fc-fusion protein. This is particularly suitable when the protein variation does not affect effector domain binding.

In case it is not known from the literature, the skilled person would know how to determine whether protein variations affect binding of the recombinant protein of interest. One way to determine the effect of protein variations on affinity ligand binding involves analyzing the relative distribution of protein variations in a sample before purification using said affinity ligand and in the eluate using the analytical separation methods as used in the methods of the invention and suitable to distinguish said variants. Alternatively, the different protein variants may be purified and the binding affinity of each variant determined separately using methods known in the art, such as by surface plasmon resonance (SPR) technology.

In another preferred embodiment the recombinant protein of interest comprises a Fc-domain and the affinity ligand is an antibody specifically binding to the specific Fc-domain, but not to endogenous antibodies in the sample. For example a human or humanized antibody, such as a human or humanized IgG antibody, may be purified from a liquid sample of a non-human mammal by using an anti-human antibody, such as an anti-human IgG antibody, as the affinity ligand. More specifically a human or humanized IgG1 antibody may be purified using an anti-human IgG1, and a human or humanized IgG2 antibody may be purified using an anti-human IgG2 antibody etc. This is particularly useful in pre-clinical studies in monkeys, rodents, dogs, cats or pigs, wherein the sample of a mammal is a monkey, a rodent, a dog, a cat or a pig sample. Preferably, the sample of a mammal is a rodent sample and the rodent sample may be, e.g., a mouse, a rat, a hamster or a rabbit sample.

Preferably, the recovery rate of the recombinant protein of interest following affinity chromatography comprising immobilizing the recombinant protein of interest on a solid support coupled to an affinity ligand specific for the recombinant protein of interest (step b) and eluting said protein of interest from the solid support (step c) is high, e.g., 75% or more, 80% or more, 85% or more, 90% or more, 95% or more, 98% or more, 99% or more or 100 %. Suitable methods to elute recombinant proteins of interest such as IgGs and fusion proteins from their affinity ligand are known to the person skilled in the art, and include without being limited thereto the use of acidic conditions, such as glycine or arginine buffer of low pH, e.g., pH < 4. Preferably the buffer is of low pH, preferably a pH of < 4, more preferably a pH of < 3.5 and even more preferably a pH of < 3. The buffer may comprise a glycine or arginine buffer, preferably a glycine buffer of a pH of < 4, more preferably a pH of < 3.5 and even more preferably a pH of < 3 and even more preferably of about pH 2.7. The buffer may further contain one or more chaotropic reagent, such as guanidinium chloride, butanol, ethanol, propanol, lithium perchlorate, lithium acetate, urea, thiourea or magnesium chloride. A preferred example of a chaotropic reagent is as guanidinium chloride at 2 to 6 M or urea at 1 to 8 M, preferably guanidinium chloride at 2 to 6 M or urea at 1 to 8 M, more preferably guanidinium chloride at about 4-6 M or urea at about 4-6 M and even more preferably guanidinium chloride at 6 mM or urea at 4 mM. In a particularly preferred embodiment the elution buffer comprises a glycine buffer of about pH 2.7 further comprising 4-6 M guanidinium chloride or 4-6 M urea and more preferably the elution buffer is a glycine buffer of about pH 2.7 comprising 6 M guanidinium chloride or 4 M urea. This buffer results in almost complete recovery of most protein-protein interactions.

Alternatively a fraction of the recombinant protein of interest may be eluted or released by enzymatic cleavage. The enzymatic cleavage according to the methods of the invention, may be done using an endoproteinase. Preferably the endoproteinase cleaves the recombinant protein efficiently and specifically (e.g., only at a known cleavage site). More preferably the enzyme is further selective for the recombinant protein of interest. This means that the specific cleavage site is rare and only present in the recombinant protein of interest and few other proteins, such as a specific family of proteins, or a group of proteins all comprising the same domain, such as Fc-containing proteins. A more selective enzyme avoids or reduces elution of fragments of non-specifically bound proteins from the solid support. For example the endoproteinases suitable in the methods of the invention to release the Fc-domain of the recombinant protein from the solid support are endoproteinase, such as papain, ficin, cysteine protease SpeB (FabULOUS) or cysteine proteinase IdeS (FabRICATOR®), preferably the endoproteinase is the cysteine proteinase IdeS (FabRICATOR®). IdeS specifically cleaves human IgG in the hinge region between the two glycines of the constant sequence ELLGGPS. Enzymatic cleavage using endoproteinases is typically very efficient, resulting in almost complete release protein fragment. Compared to acidic elution of therapeutic antibodies or fusion proteins immobilized via their respective ligand (using for example glycine of arginine), elution via enzymatic cleavage is often more efficient and complete and therefore results in more sensitivity of the method of the invention. Non-selective endoproteinases, such as Lys-C that hydrolyses specifically at the carbonyl side of Lys or trypsin, cleaving several times within most proteins are not well suited in the context of the present invention.

In fusion proteins, protein variants of the effector domain and the fusion partner, such as an Fc-domain, albumin or transferrin can be analyzed separately to gain more information about site specificity. In the following the method according to the invention will be exemplified for an Fc-fusion protein. However, the skilled artisan knows how to adapt the method to other other recombinants proteins of interest such as antibodies. For individual analysis of the Fc part and effector domain the two parts have to be separated. The fusion protein is therefore immobilized with its effector domain on a solid support, such as a sepharose resin and the Fc part is released enzymatically using an enzyme such as IdeS enzyme. IdeS is an endopeptidase which selectively cleaves IgGs and related molecules with high specificity below the hinge region producing a Fab2 and Fc fragments. IdeS also cleaves IgG Fc-domain containing fusion proteins.

The fusion protein is cleaved into an effector domain (e.g., receptor part), which is connected by disulfide bridges and able to bind the interaction partner or antigen and an Fc-domain. Alternatively any other endoproteinase could be used that cleaves selectively between the effector domain and the Fc-domain. Alternatively for other fusion proteins suitable endoproteinases cleave between the effector domain and the fusion partner, such as albumin or transferrin. Preferably, the endoproteinase only cuts the fusion protein between the effector domain and the Fc-domain. Even more preferably, the endoproteinase selectively cuts the fusion protein or a group (or family of proteins including the fusion protein) within the sample, but not all proteins within the sample. This provides further specificity of the method and reduces contamination with fragments of non-specifically bound proteins. For example, the IdeS enzyme cleaves IgG Fc-domains below the hinge region in antibodies and Fc-fusion proteins and therefore reduces contamination of the released Fc-domain by other contaminating proteins that do not have an Fc-domain. The fusion protein may also contain a specific cleavage site, which has been introduced by molecular cloning between the effector domain and the fusion partner, which may be used in this method.

To reduce heterogeneity of the affinity purified recombinant protein of interest, attached glycans may be released prior to analysis of protein variants using the analytical separating methods of the methods of the invention. Release of the glycans by enzymatic methods is preferred and causes no problems to those of skill in the art. A particularly preferred method of glycan removal for subsequent labeling (or analysis) is digestion with PNGaseF (Peptide N-glycosidase F). Various suitable PNGaseF enzymes are offered commercially under different trade names (e.g., N-Glycanase®), which are mostly engineered or optimized PNGaseF, although using an engineered or optimized PNGaseF is not mandatory in the context of the present invention. Enzymatic release may also be done by using Endoglycosidase H (or an enzyme with similar enzymatic activity, such as EndoSGlycanase IgGZERO™) or Endoglycosidase F2, which cleave between the two N-acetylglucosamines of the glycan core leaving the first monosaccharide attached to the protein. However, Endoglycosidase H and Endoglycosidase F2 are only specific for oligomannose and hybrid bi-antennary glycans.

The LC-MS used in the methods of the present invention is preferably a reverse phase LC-MS or a NanoLC-MS, more preferably the LC-MS is a reverse phase NanoLC-MS.

Methods for affinity purifying antibodies, fusion proteins or other proteins are known to the skilled person. The solid support used may be a resin, such as a resin comprising microbeads, preferably sepharose beads, agarose beads or magnetic beads, more preferably magnetic beads. However any solid support or resin suitable for affinity chromatography can be used in the methods of the present invention. The affinity ligand may be coupled to the solid support via N-hydroxysuccinimide (NHS), cyanogen bromide, epoxy, carbodiimide or thiopropyl, preferably via N-hydroxysuccinimide (NHS) or cyanogen bromide. Again, methods for coupling affinity ligands to a solid support are known in the art. Typically resins activated with one of the above linkers are commercially available.

The recombinant proteins of interest that will typically be analyzed using the methods of the present invention are therapeutically relevant recombinant proteins. Therapeutically relevant recombinant proteins often have a very high affinity and specificity to its therapeutic target. Hence the therapeutic target is well suited as an affinity ligand to capture the recombinant protein from the liquid sample of a mammal and immobilize it to the solid support. For example affinity purification of an antibody with the respective antigen has the advantage of very high affinity and specificity due to the strong interaction of the antibody with its antigen. The affinity of a therapeutic antibody for its antigen is typically in the picomolar to low nanomolar range. Therapeutically relevant fusion proteins, such as etanercept, typically bind their therapeutic target with a similar affinity. However, one major drawback of this high affinity binding is the limited recovery of antibodies or fusion proteins after acidic elution. Eluting a recombinant protein of interest from its affinity ligand may be improved by using a low pH glycine buffer of pH 4 or lower and a chaotrophic salt such as guanidinum chloride at 2-6 M or urea at 1-8 M, preferably a glycine buffer at a pH of about 2.7 comprising 4-6 M guanidinium chloride or 4-6 M urea.

The affinity ligand may be any binding partner, including without being limited thereto the ligand for a receptor, the substrate, an antigen or an antibody. For therapeutically relevant proteins the affinity ligand is preferably the therapeutic target or a fragment thereof. The affinity ligand may also be a mutant of the therapeutic target or a fragment thereof, preferably a mutant binding to the recombinant protein of interest with higher affinity compared to the wild type therapeutic target or fragment thereof. Where the recombinant protein of interest is an antibody, the affinity ligand is preferably an antigen binding to the antibody, more preferably an antigen binding to the antibody with high affinity. The antigen may be for example a complex of more than one protein, a full length protein or a fragment thereof, including a short peptide. For fusion proteins the affinity ligand may be a binding partner preferably binding to the effector domain of the fusion protein. The affinity ligand may also be an antibody binding to the effector domain of a fusion protein. For any other recombinant protein of interest, the affinity ligand may likewise be a binding partner or an antibody binding to the recombinant protein of interest. Preferably, the affinity ligand is not glycosylated.

In a preferred embodiment of the methods of the invention the two or more liquid samples of a mammal or aliquots thereof are prepared for analysis using a multi-well plate, preferably a 24, 96 or 384 well plate, more preferably a 96 well plate. When magnetic beads are used, a magnet is used to retain the beads immobilizing the recombinant protein of interest during washing. Alternatively a multi-well filter plate may be used, preferably a 24, 96 or 384 well filter plate, more preferably a 96 well filter plate. The filter membrane is preferably a low protein binding and/or hydrophile membrane, such as nitrocellulose or polyvinylidene difluoride (PVDF) membranes. These filter plates are suitable for affinity chromatography, enzymatic digest, fluorescent labeling and gel-filtration, depending on the reagents or resins added.

The liquid sample of a mammal comprising the recombinant protein of interest is a body fluid and is preferably selected from the group consisting of serum, plasma, urine, cerebral spinal fluid, amniotic fluid, saliva, sweat, ejaculate, tears, phlegm, vaginal secretion, vaginal wash and colonic wash; preferably said sample is a plasma or a serum sample. Preferably the samples to be analyzed have a volume from about 1 $\mu$l to about 1000 $\mu$l, from about 5 $\mu$l to about 500 $\mu$l, from about 10 $\mu$l to about 200 $\mu$l, from about 10 $\mu$l to about 100 $\mu$l, from about 25 $\mu$l to about 100 $\mu$l, or from about 40 $\mu$l to about 75 $\mu$l, preferably of about 50 $\mu$l. In a preferred embodiment the two or more liquid samples of a mammal were obtained from the same subject. The mammalian liquid sample analyzed in the methods of the invention may be a human, a monkey, a rodent, a dog, a cat or a pig sample, preferably a rodent sample such as from mouse, rat, hamster or rabbit. In pre-clinical studies the mammalian liquid sample is a non-human sample, preferably a monkey, a rodent, a dog, a cat or a pig sample. Further, a rodent sample may be a mouse, a rat, a hamster or a rabbit sample.

The methods of the present invention may be used to determine pharmacokinetic parameters of at least one specific protein varian of the recombinant protein of interest, preferably the $C_{max}$, $t_{max}$, AUC or $t_{1/2}$. Preferably the two or more liquid samples of a mammal were obtained at different time points from the same subject, wherein at least about 24 h, 48 h, 72 h, 96 h, 120 h, 144 h, 168 h, 336 h, 504 h, 672 h, 840 h may be between the first and the last time point. Preferably the two or more liquid samples of a mammal are 5 or more, 10 or more, 20 or more, 30 or more, 40 or more or 50 or more samples.

The methods of the invention may further comprise the steps of (i) determining the peak area of at least one of said protein variants and the internal standard in each sample (ii) normalizing the peak area of said at least one protein variant is normalized to the internal standard in each sample, and (iii) comparing the normalized peak areas from the two or more samples are compared.

The method of the invention may also comprise the generation of a standard curve in order to qualify absolute or relative protein concentrations, otherwise the comparison with the internal standard is sufficient.

For example ratios of a specific protein variant to the constant internal standard may be plotted against time result in PK profiles for each protein variant. Alternatively calculated sample protein variant percentages may be plotted against time, resulting in PK profiles of each protein variant. Determined PK profiles can be compared against the respective ELISA profile and are the basis for calculation of the protein variant distribution. Alternatively the protein concentration can be determined using a standard curve (calibration curve). The skilled person will understand that the percentages (%) or ratios of the protein variants refer to $\mu$g/ml or mg/ml, because this is the unit that the internal standard is determined using, e.g., ELISA.

The protein variants may also be analyzed according to the methods of the invention in an aliquot of each of said two or more liquid samples of a mammal. Preferably the aliquots to be analyzed have a volume from about 1 $\mu$l to about 1000 $\mu$l, from about 5 $\mu$l to about 500 $\mu$l, from about 10 $\mu$l to about 200 $\mu$l, from about 10 $\mu$l to about 100 $\mu$l, from about 25 $\mu$l to about 100 $\mu$l, or from about 40 $\mu$l to about 75 $\mu$l, preferably of about 50 $\mu$l. Optionally, the concentration of said recombinant protein of interest may be analyzed in a further aliquot of said two or more liquid samples of a mammal. In one embodiment, the concentration of said recombinant protein of interest is analyzed by ELISA or any other method known to the person skilled in the art to determine concentrations of a specific protein in a sample.

The capillary electrophoresis (CE) is an electrokinetic separation method performed in submillimeter diameter capillaries and in micro- and nanofluidic channels. In connection with the methods of the present invention preferably capillary zone electrophoresis (CZE), capillary gel electrophoresis (CGE) or capillary isoelectric focusing (CIEF) is used, more preferably capillary zone electrophoresis (CZE). CZE is preferred with the methods of the present invention as this free solution capillary electrophoresis can be connected to mass spectrometers.

The liquid chromatography-MS to be performed in connection with the methods of the present invention is preferably reverse phase liquid chromatography (RP-LC-MS), more preferably reversed-phase high performance liquid chromatography (RP-HPLC-MS), or ultra-performance liquid chromatography (UPLC-MS). In this case, as in other preferred cases of RP-LC performed in connection with the present invention, these chromatography methods are performed on a reversed-phase liquid chromatography column. Reverse phase LC is preferred in the methods of the present invention as it allows more sensitive analysis compared to normal phase LC, because the mobile phase is more compatible with mass spectrometry. Optionally, no ion-pairing reagent is used in the mobile phase. Preferably, an acidic mobile phase is used for the RP-LC.

The liquid chromatography coupled to the MS analysis to be performed in connection with the methods of the present invention is preferably a normal-flow HPLC, nano-LC or nano-HPLC, even more preferably a reverse phase HPLC, reverse phase nano-LC or a reverse phase nano-HPLC. Normal flow HPLC is characterized by an inner diameter of the columns of 1.0 mm - 2.1 mm and flow-rates of 0.050-1.000 ml/min. Nano-LC is characterized by a decreased inner diameter of the columns that are used for LC (10-150 $\mu$m) and smaller flow-rates (10-1000 nl/min) compared to conventional LC or HPLC, respectively. This down-scaling results in the ability to analyze proteinaceous samples in the low femtomole and subfemtomole ranges (Chervet et al., Analytical Chemistry 1996, 68:1507-12) and therefore allows to reduce the sample volume to be analyzed and the duration to detect protein variants of recombinant proteins of interest post administration.

A suitable mobile phase used during RP-LC comprises formic acid. In this regard, preferred amounts of formic acid in the mobile phases are from about 0.1% to about 2.0% formic acid. Typical preferred amounts therefore include about 0.2%, about 0.3%, about 0.4%, about 0.5%, about 0.6%, about 0.7%, about 0.8%, about 0.9%, about 1.0%, about 1.1%, about 1.2%, about 1.3%, about 1.4%, about 1.5%, about 1.6%, about 1.7%, about 1.8%, or about 1.9% formic acid. An amount of about 1.0% formic acid in the mobile phase is particularly preferred.

Another suitable mobile phase used during RP-LC comprises acetic acid. In this regard, preferred amounts of acetic acid in the mobile phases are again from about 0.1% to about 2.0% acetic acid. Typical preferred amounts therefore include about 0.2%, about 0.3%, about 0.4%, about 0.5%, about 0.6%, about 0.7%, about 0.8%, about 0.9%, about 1.0%, about 1.1%, about 1.2%, about 1.3%, about 1.4%, about 1.5%, about 1.6%, about 1.7%, about 1.8%, or about 1.9% acetic acid. An amount of about 1.0% acetic acid in the mobile phase is particularly preferred.

Further, suitable pH values of the mobile phase used during RP-LC are in the range of about 1 to about 4, more preferably in the range of about 1.5 to about 3, yet more preferably of about 1.8 to about 2.9, even more preferably of about 1.9 to about 2.75, and particularly preferably of about 2 to about 2.7. Preferred is in particular a pH value in the range of about 2.1 to about 2.18. Accordingly, preferred mobile phases used during RP-LC in accordance with the methods and uses described and/or claimed herein will have a pH value of about 1.9, 2, 2.1, 2.2, 2.3, 2.4, 2.5, 2.6, or 2.7, with pH values of about 2.1 or of about 2.18 being particularly preferred.

The high-performance liquid chromatography (HPLC) to be performed in connection with the methods of the present invention is preferably a reverse phase or nano-HPLC, even more preferably a reverse phase nano-HPLC. Nano-LC is characterized by a decreased inner diameter of the columns that are used for HPLC (10-150 $\mu$m) and smaller flow-rates (10-1000 nl/min) compared to conventional HPLC, respectively. This down-scaling results in the ability to analyze proteinaceous samples in the low femtomole and subfemtomole ranges (Chervet et al., Analytical Chemistry 1996, 68:1507-12) and therefore allows to reduce the sample volume to be analyzed and the duration to detect protein variants of recombinant proteins of interest post administration.

In the course of the methods of the present invention the separation of the protein variant by LC, HPLC, RP-LC or RP-HPLC may be performed at temperatures in the range of about 4°C to about room temperature, or at room temperature (with room temperature being defined as 23°C for the purpose of the present invention). Preferably, however, the separation is performed at temperatures above room temperature. Preferred in this regard is a temperature in the range of about 30°C to about 100°C, about 40°C to about 80°C, about 40°C to about 60°C, with about 50°C being particularly preferred.

Suitable flow rates for a nanoRP-LC or nanoLC column in accordance with the present invention will be readily known or determined by those of skill in the art. Generally, suitable flow rates will typically be in a range of about 50-1000 nl per minute. Preferred are, for example, flow rates in a range of about 300-700 nl per minute. Accordingly, preferred values for the flow rate are about 300 nl per minute, about 400 nl per minute, about 500 nl per minute, about 600 nl per minute, or about 700 nl per minute, with a flow rate of about 300 nl per minute being particularly preferred.

Suitable flow rates for a normal flow HPLC or HPLC column with, e.g., 1.0 mm inner diameter in accordance with the present invention will be readily known or determined by those of skill in the art. Generally, suitable flow rates will typically be in a range of about 0.050-0.300 ml per minute. Preferred are, for example, flow rates in a range of about 0.050-0.200 ml per minute. Accordingly, preferred values for the flow rate are about 0.050 ml per minute, about 0.100 ml per minute, about 0.150 ml per minute or about 0.200 ml per minute, with a flow rate of about 0.100 ml per minute being particularly preferred.

Suitable flow rates for a normal flow HPLC or HPLC column with, e.g., 2.1 mm inner diameter in accordance with the present invention will be readily known or determined by those of skill in the art. Generally, suitable flow rates will typically be in a range of about 0.100-1.000 ml per minute. Preferred are, for example, flow rates in a range of about 0.100-0.500 ml per minute. Accordingly, preferred values for the flow rate are about 0.100 ml per minute, about 0.200 ml per minute, about 0.300 ml per minute, about 0.400 ml per minute or about 0.500 ml per minute, with a flow rate of about 0.300 ml per minute being particularly preferred.

In a preferred embodiment the methods of the invention allow for analyzing attomolar concentrations of the individual protein variants, e.g., concentrations as low as 800 amol, preferably as low as 600 amol, and more preferably as low as 400 amol. In another preferred embodiment the method allow for analyzing the protein variants of the recombinant protein of interest at a recombinant protein concentration in each of the two or more liquid samples of a mammal of 20 $\mu$g/ml or less, 10 $\mu$g/ml or less, 5 $\mu$g/ml or less, 2 $\mu$g/ml or less, 1 $\mu$g/ml or less, 0.5 $\mu$g/ml or less, 0.2 $\mu$g/ml or less or 0.1 $\mu$g/ml or less. In yet another preferred embodiment the methods of the present invention allow for analyzing the protein variants of the recombinant protein of interest in the two or more liquid samples of a mammal comprising 1.0 $\mu$g or less, 0.5 $\mu$g or less, 0.25 $\mu$g or less, 0.1 $\mu$g or less, 0.05 $\mu$g or less, 0.025 $\mu$g or less, 0.01 $\mu$g or less, or 0.005 $\mu$g or less of the recombinant protein of interest each. In yet another preferred embodiment at least steps a), b) and c) are operated in a high throughput manner.

In a preferred embodiment the recombinant protein of interest is affinity purified using 96-well and small volume samples (e.g. 50 $\mu$l, 25 $\mu$l or less) of preclinical or clinical serum samples. Preferably the recombinant protein of interest is a monoclonal antibody or a fusion protein and the affinity ligand immobilized on the solid support is the antigen of the monoclonal antibody, an antibody or the ligand or receptor specifically binding to the fusion protein. The affinity ligand, such as the antigen or antibodies, is highly specific for the biopharmaceutical immobilized covalently to protein reactive beads (e.g. beads carrying NHS groups), preferably protein reactive magnetic beads. Beads with immobilized antigen or antibody are added to serum samples together with an internal standard and incubated for a time period sufficient for binding, in a 96-well plate. The internal standard also binds the affinity ligand, such as the antigen or an antibody. Beads are separated from the sample for example by filtration using filter plates or if the beads are magnetic beads they can be collected at the wall of each well using a magnet. Serum samples can be removed and the beads with the immobilized internal standard and recombinant protein of interest be washed to remove unspecifically bound serum proteins. Finally the internal standard and the target protein are eluted from the beads using a pH shift and high concentrations of chaotropic reagent to fully recover the analyte, i.e., the protein variant(s) of the recombinant protein of interest, and the internal standard. Various modifications or protein variants of the purified recombinant protein of interest and the internal standard standard can then be analyzed by analytical separating methods, such as (nano)HPLC, (nano)LC, (nano)LC-MS, MS or capillary electrophoresis methods after additional sample preparation steps depending on the variant to be analyzed (e.g. enzymatic digest, deglycosylation, reduction, etc.). Based on the ratio of the sample and constant standard signal intensities a pharmacokinetic profile is obtained for each modification or variant individually.

The invention is further illustrated by the following Figures and Examples, which are not to be considered as being limiting for the scope of protection conferred by the claims of the present application.

### *Examples*

#### Materials

Buffers and chemical used in the following experiments are disodium hydrogen phosphate dehydrate (Merck; Cat. No. 1.06580.1000), DMSO (Sigma-Aldrich; Cat. No. 41647), D-PBS (Gibco/Invitrogen/Life; Cat. No. 14190094), D-PBS powder (Gibco/Invitrogen/Life, Cat. No. 21600069), ethanolamine (Sigma-Aldrich; Cat. No. 398136), glycine (Merck; Cat. No. 5.00190.1000), guanidinium chloride (Merck, Cat. No. 1.04220.1000), 1 N hydrochloric acid (Merck, Cat. No. 1.09057.1000), 25% hydrochloric acid (Merck; Cat. No. 1.00312.2500), NHS-magnetic beads (Pierce Biotech / Thermo Scientific; Cat. No. 88827), potassium chloride (Carl-Roth; Cat. No. P017.2), potassium dihydrogen phosphate (Merck; Cat. No. 1.04873.1000), sodium chloride (Merck; Cat. No. 1.06404.1000), trifluoracetic acid (for spectroscopy) (Merck; Cat. No. 1.08262.0025), TRIS (Merck; Cat. No. 1.08386.1000), 1 M TRIS HCl pH= 8.0 (Gibco/Invitrogen/Life, Cat. No. 15568-025), acetonitrile (for HPLC) (Merk; Cat. No. 1.00030.2500), formic acid (Fluka; Cat. No. 94318), NZW rabbit serum (Sera Laboratories), TCEP (Calbiochem; Cat. No. 580560) and urea (Sigma Aldrich, Cat. No. U4884).

**Example 1:** PK profiling of disulfide isoforms of an IgG1 antibody

#### Preclinical rabbit study

The preclinical study was performed in cynomolgus monkeys. Following single subcutaneous administration of 3 mg kg-1 body weight of an IgG2 mAb1, blood samples were drawn over a period of time including one pre-dose blood sample. Serum samples were taken at 10 time points at 8, 24, 48, 60, 72, 96, 120, 192, 240 and 336 hours post administration. Concentration of mAb1 in serum was determined by ELISA. From remaining serum 2 x 50 $\mu$l aliquots were used for PK profiling. The first aliquot was analyzed and the second aliquot served as back-up aliquot.

#### Buffer preparation

Acidification solution , 1 mM hydrochloric acid pH 3.0: 100 $\mu$l of 1 N (1 M) hydrochloric acid were pipetted into a 100 ml bottle filled with 99.8 g ultra-pure water. After briefly mixing the pH was measured and should be 3.0. The solution has to be prepared fresh before use.

Blocking buffer, 0.5 M ethanolamine, 0.5 M NaCl pH 8.3: 2.92 g NaCl and 3.02 ml ethanolamine were dissolved in ~80 ml ultra-pure water. With the use of 25% hydrochloric acid the pH was adjusted to 8.3. Buffer was filled up to 100 ml with ultra-pure water. The solution is stable at room temperature for at least 4 weeks. pH should be checked before use.

Elution buffer, 0.1 M glycine pH 2.7 with 6 M guanidinium chloride: To obtain a 1 M glycine stock solution, 7.5 g glycine were dissolved in ~80 ml ultra-pure water. pH was adjusted to 2.7 with use of 25% hydrochloric acid and filled up to 100 ml with ultra-pure water. The solution is stable at room temperature for at least 4 weeks. 10 ml of the 1 M glycine stock solution were mixed with 57.3 g guanidinium chloride and filled up to 100 ml with ultra-pure water and the pH checked before use. The solution is stable at room temperature for at least 4 weeks

Mobile phase A (RP-C4 HPLC), 80% ultra-pure water, 10% 2-propanol, 10% acetonitrile with 0.1%: TFA: 798.4 g ultra-pure water, 78.6 g 2-propanol and 78.2 g acetonitrile were weight in a 1 l bottle on a balance. 1.0 ml trifluoroacetic acid was pipetted into the provided solvents and mixed well. The mobile phase is stable for 1 month and must be exchanged regularly to ensure stable chromatographic conditions.

Mobile phase B (RP-C4 HPLC), 30% ultra-pure water, 10% 2-propanol, 60% acetonitrile with 0.1% TFA: 299.4 g ultra-pure water, 78.6 g 2-propanol and 469.2 g acetonitrile were weight in a 1 l bottle on a balance. 1.0 ml trifluoroacetic acid was pipetted into the provided solvents and mixed well. The mobile phase is stable for 1 month and must be exchanged regularly to ensure stable chromatographic conditions.

Reconstitution of the antigen

Recombinant human antigen specifically binding to IgG2 mAb1 produced in E.coli was reconstituted according to the manufacturer instructions. Antigen was dissolved in $H_2O$ (1 mg/mL) and reconstituted for 2 hours at room temperature.

Preparation of Fc-fusion protein as internal standard.

A Fc fusion protein (containing the natural receptor of the antigen) capable of binding the same antigen as the sample antibody IgG2 mAb1 served as internal standard for the affinity purification and disulfide analysis by HPLC. The standard was reconstituted in PBS at 100 μg/ml and was subsequently aliquoted a 5 μg in 0.25 ml vials, frozen in liquid nitrogen and stored until use at -70°C.

200 μl PBS were added to each tube to furnish a concentration of 20 μg/ml. All prepared standards were mixed before use. 55 μl of the solution were added to serum samples during sample preparation to add 1.05 μg of the internal standard to each sample.

Magnetic bead preparation and antigen coupling

Per sample 24.5 μl of NHS magnetic beads (10mg/ml in DMAc) and 5 μg antigen were used. Beads were pipetted into a new 1.5 ml tube. As the magnetic beads are moisture-sensitive the bottle was immediately capped after removing the slurry in order to protect the beads and wrapped with lab film around the cap before storing at 4°C. 500 μl 1 mM hydrochloric acid were added and the tube was placed into the DynaMag™-2 magnet to collect the beads at the tube wall. Solution was carefully aspirated and discarded. The beads were washed four times with 500 μl 1 mM hydrochloric acid and solution was carefully aspirated and discarded. Antigen solution (0.25 mg/ml in PBS; 20μl per sample) was added to the beads and incubated for 2 hours at ambient temperature in an overhead shaker. The tube was placed into the DynaMag™-2 magnet to collect the beads at the tube wall and the coupling solution was carefully aspirated. Magnetic beads were washed and deactivated with 500 μl blocking buffer for two times. Blocking reaction was allowed to take place for 5 minutes at ambient temperature in an overhead shaker. The tube was placed again into the DynaMag™-2 magnet to collect the beads at the tube wall and the coupling solution was carefully aspirated. Finally, beads were equilibrated four times with 20μl PBS per sample. Again, liquid was removed using the magnet. In case more than one preparation was needed, the coupled and equilibrated beads were pipetted together and mixed carefully. If prepared beads were not used immediately they were stored in PBS at +4°C until use (maximum 7 days).

Affinity purification of the protein variants of IgG2 mAb1

A constant amount of internal standard (antigen-Fc) was spiked into serum samples. IgG2 mAb1 and internal standard were recovered from serum samples using the respective recombinant human antigen immobilized on magnetic beads. Following antigen binding and washing intact IgG2 mAb1 was eluted using an acidic buffer containing a chaotropic reagent. The eluate was neutralized and subsequently analyzed by reversed phase (C4) chromatography which is able to separate different disulfide variants. Changes in the disulfide pattern of IgG2 mAb during circulation and a potential impact on the PK can be measured.

The serum samples were aliquoted in portions of 55 μl and were thawed in the refrigerator overnight. The 55 μl of serum sample were diluted with 55 μl of the 20 μg/ml internal standard solution in PBS. 100 μl of the samples were transferred into a PP V-bottom 96-well plate, which is compatible to 96-well magnet type A. 20 μg of magnetic beads with immobilized antigen were added to each sample. The mixture was incubated at ambient temperature at 1200 rpm for 1 hour in a 96-well plate shaker / heater and beads were washed five times with 200 μl PBS each. The liquid was removed each time using the 96-well magnet type A. 200 μl of elution buffer were added to the beads and incubated at ambient temperature for 5 minutes, before the plate was placed into the magnet device to collect the beads at the well wall and the solution was carefully aspirated and transferred into a new 96-well plate. The beads were washed again with 50 $\mu$l of elution buffer and this solution was also transferred to the 96-well plate and mixed with 25 $\mu$l of 1 M TRIS to neutralize, resulting in a total volume of 275 $\mu$l that contains the released intact antibody. Purified samples were analyzed by HPLC on a C4 column.

HPLC analysis of intact protein

The neutralized eluate was analyzed by reverse phase (C4) chromatography, which is able to separate different disulfide variants. Changes of the disulfide pattern of IgG2 mAb1 during circulation and the potential impact on the pharmacokinetics were measured using mobile phase A and B and the HPLC parameters according to table 2.

**Table 2**

| Injector | | |
|---|---|---|
| Auto sampler temperature | 2-8°C | |
| Injection volume | 100 $\mu$l | |
| **Column heater** | | |
| Temperature | 80.0 C | |
| Column | BIOshell A400 ProteinC4; 15 cm x 2,1 mm; 3,4$\mu$m (Supelco; 66826-U) | |
| **Pump** | | |
| Flow rate | 0.3 ml/min | |
| Run time | 23.0 min | |
| Max. pressure | 400 bar | |
| Typical max. working pressure | 100 bar | |
| Gradient | Time (min) | Mobile phase B (%) |
| | 0 | 15 |
| | 2 | 15 |
| | 7 | 25 |
| | 32 | 35 |
| | 33 | 95 |
| | 38 | 95 |
| | 39 | 15 |
| Column equilibration time | 4.0 min | |
| **Detector (UV)** | | |
| Detection (DAD) | Detection: 214; 206; 280 nm | |
| | 10 mm flow cell | |
| | peak width > 0.1 min (2s) | |
| Detection (FLD) | Ex: 280 nm; Em: 348 nm; PmtGain 15 | |
| | Peakwidth 0.2 min; polarity + | |
| | Flashrate Standard | |

Raw data were processed at UV (214 nm) and the FLD (ex280nm; em348nm) chromatograms were integrated manually. The resulting peak area values were used to calculate the ratio of IgG2 mAb1 peak to Fc-fusion protein peak ($P_x$/R). For each of the 5 different IgG2 peaks the ratio was calculated according to equation 1.

Equation 1: $P_{(1-5)}/R$ = Peak Area IgG mAb1/Peak Area internal standard

With the determined $P_x/R$ and the know relationship of the Fc-fusion internal standard, determined in the same sequence, the relative disulfide composition can be calculated at each time point using Equation 2.

$$\text{Equation 2: } \%P_x = \frac{P_x Peak\ area}{\sum_{P_1}^{P_5} Peak\ area}$$

Selective clearance of disulfide isoforms

Peak to internal standard ratios and AUCs were calculated and statistical analysis (unpaired t-test & test for equal variances) was performed with the obtained AUCs.

In order to obtain PK profiles, the five peaks were normalized to the highest relative concentration of each profile (concentration-time curve) to compare the profiles of the different disulfide isoform. These normalized PK profiles are shown in Figure 3. AUCs were calculated from the normalized PK profiles from each animal and peak.

Equivalence and 2-sample t-tests were performed to test if the PK profiles are statistically equivalent, different or inconclusive. Equivalence testing of P2, P3, P4 and P5 was performed against the main variant P1 (Figure 4). Profiles of P1 and P2 are equivalent. Profiles of P1 and P3, P1 and P4 and P1 and P5 are not equivalent.

2-sample t-tests (alpha 0.05) were performed again comparing the main variant P1 against P2, P3, P4 and P5. Resulting p-values are listed in Table 1. Profiles of P1 and P2 are not statistically different (p=0.882), which is in agreement with the results from equivalence testing. PK profiles of P1 and P3 and P1 and P5 are statistically different with p-values of 0.002 and 0.037. Unpaired t-test of P1 and P4 is not statistically significant with p=0.055 and remains inconclusive.

**Table 3.** Results of the t-tests

| Unpaired t-test | P1-P2 | P1-P3 | P1-P4 | P1-P5 |
|---|---|---|---|---|
| p-value | 0.882 | 0.002 | 0.055 | 0.037 |

**Example 2:** PK profiling of N-terminal leader peptide extensions on IgG1 light chains

Antibodies can contain variations at their N-termini of both heavy and light chain. These extensions can result from incomplete processing during protein biosynthesis. The extension can for example contain charged or hydrophobic amino acids that can influence the physico-chemical properties (e.g. binding of the antigen). In the present case a portion of the IgG1 biopharmaceutical has an N-terminal leader peptide extension on one light chain. The affinity purification work-flow is illustrated in Figure 5.

Preclinical rabbit study

The preclinical study was performed in Himalayan rabbits following intraveneous administration of 15 mg/kg body weight over 30 minutes of a humanized IgG1 mAb2 antibody. The mAb2 antibody contains to some extend an N-terminal leader peptide extension on the light chain. The blood samples were drawn over a period of time including one pre-dose blood sample. Sampling was performed 10, 20 and 30 minutes after infusion start and 40, 50, 60 2, 4, 8, 24, 48, 72, 96, 120 and 144 hours post administration.

Buffer preparation

Acidification solution, 1 mM hydrochloric acid pH 3.0: 100 $\mu$l of 1 N (1 M) hydrochloric acid were pipetted into a 100 ml bottle filled with 99.8 g ultra-pure water. After briefly mixing the pH was measured and should be 3.0. The solution has to be prepared fresh before use.

Elution buffer, 0.1 M glycine pH 2.7 with 4 M urea: To obtain a 1 M glycine stock solution, 7.5 g glycine were dissolved in ~80 ml ultra-pure water and the pH was adjusted to 2.7 with use of 25% hydrochloric acid and filled up to 100 ml with ultra-pure water. The solution is stable at room temperature for at least 4 weeks. 10 ml of the 1 M glycine stock solution were mixed with 24.0 g urea, filled up to 100 ml with ultra-pure water and the pH was be checked before use. The solution is stable at room temperature for at least 4 weeks

Phosphate buffered saline pH 8.5: Adjust pH of PBS pH 7.3-7.5 to pH 8.5 using $NH_4OH$.

TCEP solution, 50 mg/ml TCEP in 100 mM Tris-HCl pH 7.4: Commercially available 1 M TRIS-HCl pH 8.0 (e.g. from Gibco, #15568-025) was used and the pH was adjusted using 1 M HCl.

Mobile phase A (nanoLCMS), ultra-pure water with 0.1% FA; 5% CAN: 100 μl formic acid and 5 ml acetonitrile were pipetted into 95 ml ultra-pure water and mixed thoroughly. The mobile phase is stable for 14 days and must be exchanged regularly to ensure stable chromatographic conditions.

Mobile phase B (nanoLCMS), 95% Acetonitrile with 0.1% TFA: 100 μl formic acid and 5 ml ultrapure water were pipetted into 95 ml acetonitrile and mixed thoroughly. The mixture was degassed using ultrasonication for 15 minutes. The mobile phase is stable for 14 days and must be exchanged regularly to ensure stable chromatographic conditions.

Mobile phase loading pump (nanoLCMS); 99% ultra-pure water, 1% acetonitrile, 0.1% TFA: 1 ml acetonitrile and 100 μl TFA were pipetted into 99 ml ultra-pure water and mixed thoroughly. The mixture was degassed using ultrasonication for 15 minutes. The mobile phase is stable for 14 days and must be exchanged regularly to ensure stable chromatographic conditions.

Sample material

Preclinical serum samples were thawed, briefly mixed and 25 μl were mixed with 25 μl PBS in a 96-well plate. Affinity purification was performed immediately after serum sample preparation.

Preparation of the IgG1 internal standard

An human IgG1 antibody (~20mg/ml) was used as internal standard for relative quantification. A dilution with a concentration of 1 mg/ml was prepared by mixing 48 μl IgG1 with 952 μl NZW rabbit serum. This serum solution was further diluted to a final concentration of 0.1 mg/ml with PBS.

Calibration curve

A dilution series of the sample antibody IgG1 mAb2 in NZW serum was used as a calibration curve on each 96-well plate. The calibration curve was prepared as described in Table 4. The 1 mg/ml sample antibody was prepared by mixing 33 μl IgG1 mAb2 (sample antibody) with 967 μl NZW rabbit serum. The calibration curve samples can be stored at 2-8°C for two weeks or quick frozen in liquid nitrogen and stored < -70°C for one year.

**Table 4** Calibration curve preparation

| Concentration | μl Serum | μl sample antibody (1 mg/ml) | μl internal standard (0.1 mg/ml) |
|---|---|---|---|
| Blank | 250 | - | 250 |
| 10 μg/ml | 247.5 | 2.5 | 250 |
| 25 μg/ml | 237.5 | 12.5 | 250 |
| 50 μg/ml | 232.5 | 17.5 | 250 |
| 100 μg/ml | 225 | 25 | 250 |
| 200 μg/ml | 200 | 50 | 250 |
| 300 μg/ml | 175 | 75 | 250 |
| 400 μg/ml | 150 | 100 | 250 |

A 1:1 (mol/mol) mixture of internal standard and sample antibody with a concentration of 0.1 mg/ml was prepared as reference sample and injected prior and after each sequence into the LC-MS to ensure proper function. The mixture was prepared by mixing 4.8 μl internal standard (~20 mg/ml) with 3.3 μl sample antibody (30 mg/ml) and 992 μl PBS. 25 μl of this mixture were diluted with 50 μl elution buffer in a separate well of the 96-well plate when samples are eluted.

Magnetic bead preparation and antigen coupling

Affinity magnetic beads were prepared in 1.5 ml reaction tubes. In case the volume of magnetic beads was below 1 ml, the coupling reaction was performed in a single tube. In case the volume of the magnetic beads exceeded 1 ml the coupling was performed in two or more tubes. For each affinity purification, 26 μg of anti-human-IgG IgG solution and 100 μl NHS magnetic beads (1 mg) solution are required.

Per sample 100 μl of NHS magnetic beads (10 mg/ml in DMAc) were used. Beads were pipetted into a new 1.5 ml tube. As the magnetic beads are moisture-sensitive the bottle was immediately capped after removing the slurry in order to protect the beads and wrapped with lab film around the cap before storing at 4°C. The tube containing the magnetic beads was placed into the DynaMag™-2 magnet to collect the beads at the tube wall. DMAc storage solution was carefully aspirated and discarded. 500 µl 1 mM hydrochloric acid were added and the tube was placed into the DynaMag™-2 magnet to collect the beads at the tube wall, before the solution was carefully aspirated and discarded. The beads are washed four times with 500 µl 1 mM hydrochloric acid and solution was carefully aspirated and discarded. The anti-human-IgG IgG solution (2.3 µl per sample; diluted with 97.7 µl PBS per sample respectively) was added to the beads and incubated for 2 hours at 20°C in a thermomixer at 1000 rpm. The tube was placed into the DynaMag™-2 magnet to collect the beads at the tube wall and the coupling solution was carefully aspirated. Magnetic beads were washed and deactivated with 500 µl blocking buffer (1 M TRIS-HCI pH 7.4) for two times. Blocking reaction was allowed to take place for 5 minutes at ambient temperature in a thermomixer at 1000 rpm. The tube was placed again into the DynaMag™-2 magnet to collect the beads at the tube wall and the coupling solution was carefully aspirated. Beads were equilibrated four times with 50µl PBS per sample. Again, liquid was removed using the magnet. Finally beads were suspended in 50 µl PBS per sample. In case prepared beads were not used immediately they can be stored in PBS at +4°C until use (maximum 7 days).

Affinity purification of the human IgG antibody

25 µl per sample of the IgG 1 internal standard antibody (0.1 mg/ml) were pipetted to the serum samples in a 96-well plate. Calibration curve samples (25 µl each) were pipetted into row 1 of each 96-well plate. Magnetic beads with immobilized anti-human IgG (50 µl/sample) were added to the prepared serum samples and calibration curve samples in a 96-well plate and incubated at ambient temperature for 1 hour in a 96-well plate shaker / heater at 1200 rpm. Beads are washed six times; twice with 200 µl PBS each; three times with 200 µl PBS pH 8.5 and finally once with 200 µl PBS. After each PBS addition the plate shortly mixed at 1200 rpm in a 96-well plate shaker / heater. Liquid was removed each time using the 96-well magnet type A. 50 µl of elution buffer were added to the beads and incubated at ambient temperature for 5 minutes before the plate was placed into the magnet device to collect the beads at the well wall and the solution containing the released intact antibodies (sample antibody and internal standard) was carefully collected and transferred into a new 96-well plate. 25 µl of the solution containing the sample antibody/internal standard reference were mixed with 50 µl elution buffer in an empty well. Per well 10 µl of a TCEP solution (0.5 mg/ml in 0.1 M Tris HCI) were added to the antibody solution and incubated at ambient temperature for 30 minutes at a 96-well plate shaker / heater at 1200 rpm. Samples were analyzed by RP nanoLC-MS. The ratios of MS intensities (Biopharmaceuticals/Internal standard and Biopharmaceutical with extension/Internal standard) were extracted from MS spectra. A calibration curve obtained from spiked serum samples with constant internal standard and a dilution series of the biopharmaceutical was used to determine the relative concentration of biopharmaceutical and leader peptide extension. Exemplarily shown in Figure 6. N-terminal heterogeneity of IgG1 mAb2 during circulation and the potential impact on the pharmacokinetics were measured using mobile phase A and B and the parameters according to table 5.

**Table 5**

| Injector | |
|---|---|
| Auto sampler temperature | 2-8°C |
| Injection volume | 10-15 µl |
| **Column heater** | |
| Temperature | 60.0 C |
| Column | Pepswift trapping column (Thermo Scientific 164558); Pepswift analytical column (Thermo Scientific 164584) |
| **Pump** | |
| Flow rate | 0.700 µl/min |
| Run time | 16.0 min |
| Max. pressure | 800 bar |
| Typical max. working pressure | 200 bar |

(continued)

| Pump | | |
|---|---|---|
| | Time (min) | Mobile phase B (%) |
| | 0 | 5 |
| | 2 | 5 |
| | 3 | 25 |
| Gradient | 10 | 35 |
| | 11 | 95 |
| | 12 | 95 |
| | 13 | 5 |
| | 16 | 5 |
| | Time (min) | Valve setting |
| | 0 | 1_2 |
| Valve position | 2 | 1_6 |
| | 15 | 1_2 |

Results

The results of the study are shown in Figure 7 indicating that there is no difference in PK between leader peptide and non-leader peptide containing antibody for the investigated biopharmaceutical.

## Claims

1.    A method of analyzing protein variants of a recombinant protein of interest in liquid samples of a mammal comprising

a) providing two or more liquid samples of a mammal comprising the recombinant protein of interest;
b) immobilizing the recombinant protein of interest of each of said samples on a separate solid support coupled to an affinity ligand specific for the recombinant protein of interest in the samples;
c) eluting the recombinant protein of interest or a fragment thereof of each of said samples from the solid support into separate eluates; and
d) analyzing the protein variants of step c) of each of said samples separately using an analytical separating method and comparing said two or more samples,

wherein an internal standard binding to the same affinity ligand is added to each of said samples prior to step b and the internal standard is analyzed together with the recombinant protein of interest of step d), and
wherein the protein variants of the recombinant protein of interest to be analyzed are due to deamidation, oxidation, N-terminal heterogeneity, C-terminal heterogeneity, isomerization, or disulfide isoforms.

2.    The method of claim 1, wherein the method is for analyzing one or more pharmacokinetic parameter of said protein variants.

3.    The method of claim 2, wherein the analytical separating method is suitable to distinguish said protein variants of the recombinant protein of interest, preferably selected from the group consisting of HPLC, capillary electrophoresis (CE) or mass spectrometry (MS).

4.    The method of claim 2, wherein

(i) protein variants of the recombinant protein of interest are due to deamidation, oxidation, N-terminal heterogeneity, C-terminal heterogeneity, or isomerization and the analytical separating method is selected from the group consisting of HPLC, CE and MS; or

(ii) protein variants of the recombinant protein of interest are due to disulfide isoforms and the analytical separating method is selected from the group consisting of HPLC and CE.

5. The method of any one of the preceding claims, wherein the internal standard binds to the affinity ligand with an affinity comparable to the recombinant protein of interest and is distinguishable from the recombinant protein of interest using said analytical separating method.

6. The method of any one of the preceding claims, wherein the recombinant protein of interest is a fusion protein or an antibody, preferably a Fc-fusion protein or an antibody.

7. The method of claim 6, wherein the recombinant protein of interest is

(i) an antibody, and the variable region of the antibody binds to the affinity ligand immobilized on the solid support, wherein the protein variation does not affect binding of said antibody to its ligand and the affinity ligand is an antigen;
(ii) a Fc-fusion protein comprising a Fc-domain and an effector domain and the effector domain binds to the affinity ligand immobilized on the solid support, wherein the affinity ligand is a binding partner or an antibody specifically binding to the effector domain of the Fc-fusion protein; or
(iii) a Fc-fusion protein or an antibody and the affinity ligand binds to the Fc domain of the recombinant protein of interest, but not to endogenous antibodies in the sample.

8. The method of claim 6 or 7, wherein the recombinant protein of interest is an IgG antibody, preferably an IgG1 or IgG2 antibody, more preferably a human or humanized IgG1 or IgG2 antibody.

9. The method of any one of claims 3 to 8, wherein

(i) the analytical separating method is MS, preferably coupled to HPLC or CE, more preferably LC-MS, and even more preferably NanoLC-MS;
(ii) the solid support is a resin comprising microbeads, preferably sepharose beads, agarose beads or magnetic beads, more preferably magnetic beads;
(iii) the affinity ligand is coupled to the solid support via N-hydroxysuccinimide (NHS), cyanogen bromide, epoxy, carbodiimide or thiopropyl, preferably via N-hydroxysuccinimide (NHS); and/or
(iv) the two or more liquid samples of a mammal are prepared for analysis in a multi-well plate, preferably in a 24, 96 or 384 well plate, more preferably in a 96 well plate, preferably a multi-well filter plate.

10. The method of any one of the preceding claims, wherein the liquid samples of a mammal are body fluids, preferably selected from the group consisting of serum, plasma, urine, cerebral spinal fluid, amniotic fluid, saliva, sweat, ejaculate, tears, phlegm, vaginal secretion, vaginal wash and colonic wash; preferably wherein said samples are plasma or serum samples.

11. The method of any one of the preceding claims, wherein one or more pharmacokinetic parameters of at least one specific protein variant of the recombinant protein of interest are determined, preferably the $C_{max}$, $t_{max}$, AUC or $t_{1/2}$.

12. The method of any one of the preceding claims, wherein the protein variants are analyzed in an aliquot of each of said two or more liquid samples of a mammal and optionally the concentration of said recombinant protein of interest is analyzed in a further aliquot of said two or more liquid samples of a mammal.

13. The method of any one of the preceding claims, wherein the samples or the aliquots to be analyzed have a volume from about 1 µl to about 1000 µl, from about 5 µl to about 500 µl, from about 10 µl to about 200 µl, from about 10 µl to about 100 µl, from about 25 µl to about 100 µl, or from about 40 µl to about 75 µl, preferably of about 50 µl.

14. The method of any one of the preceding claims, wherein the mammalian liquid sample is

(i) a human, a monkey, a rodent, a dog, a cat or a pig sample; or
(ii) a non-human sample, preferably a monkey, rodent, dog, cat or pig sample;

wherein a rodent sample is preferably a mouse, a rat, a hamster or a rabbit sample.

**Patentansprüche**

1. Verfahren zur Analyse von Proteinvarianten eines rekombinanten Proteins von Interesse in flüssigen Proben eines Säugetiers, umfassend:

   a) Bereitstellen von zwei oder mehr flüssigen Proben eines Säugetiers, die das rekombinante Protein von Interesse enthalten;

   b) Immobilisieren des rekombinanten Proteins von Interesse für jede der Proben auf einem separaten festen Träger, der mit einem Affinitätsliganden gekoppelt ist, der spezifisch für das rekombinante Protein von Interesse in den Proben ist;

   c) Eluieren des rekombinanten Proteins von Interesse oder eines Fragments davon von jeder der Proben vom festen Träger in separate Eluate; und

   d) Analysieren der Proteinvarianten von Verfahrensschritt c) jede der Proben getrennt voneinander, unter Verwendung eines analytischen Trennverfahrens sowie Vergleichen der zwei oder mehreren Proben, wobei ein interner Standard, der an den gleichen Affinitätsliganden b bindet, zu jeder der Proben vor Verfahrensschritt b hinzugefügt wird, wobei der interne Standard zusammen mit dem rekombinanten Protein von Interesse von Verfahrensschritt d) analysiert wird, und wobei die Proteinvarianten des rekombinanten Proteins von Interesse aufgrund von Desamidierung, Oxidation, N-terminaler Heterogenität, C-terminaler Heterogenität, Isomerisierung oder Disulfidisoformen für die Analyse vorliegen.

2. Verfahren nach Anspruch 1, wobei das Verfahren zum Analysieren eines oder mehrerer pharmakokinetischer Parameter der Proteinvarianten dient.

3. Verfahren nach Anspruch 2, wobei das analytische Trennverfahren geeignet ist, die Proteinvarianten des rekombinanten Proteins von Interesse zu unterscheiden, vorzugsweise ausgewählt aus der Gruppe bestehend aus HPLC, Kapillarelektrophorese (CE) oder Massenspektrometrie (MS).

4. Verfahren nach Anspruch 2, wobei

   (i) Proteinvarianten des rekombinanten Proteins von Interesse auf Deamidierung, Oxidation, N-terminale Heterogenität, C-terminale Heterogenität oder Isomerisierung zurückzuführen sind und das analytische Trennverfahren aus der Gruppe bestehend aus HPLC, CE und MS ausgewählt ist; oder

   (ii) Proteinvarianten des rekombinanten Proteins von Interesse auf Disulfidisoformen zurückzuführen sind und das analytische Trennverfahren aus der Gruppe bestehend aus HPLC und CE ausgewählt ist.

5. Verfahren nach einem der vorhergehenden Ansprüche, wobei der interne Standard an den Affinitätsliganden mit einer Affinität bindet, die mit dem rekombinanten Protein von Interesse vergleichbar ist und sich unter Verwendung des analytischen Trennverfahrens von dem rekombinanten Protein von Interesse unterscheiden lässt.

6. Verfahren nach einem der vorhergehenden Ansprüche, wobei das rekombinante Protein von Interesse ein Fusionsprotein oder ein Antikörper ist, vorzugsweise ein Fc-Fusionsprotein oder ein Antikörper.

7. Verfahren nach Anspruch 6, wobei das rekombinante Protein von Interesse

   (i) ein Antikörper ist und die variable Region des Antikörpers an den auf dem festen Träger immobilisierten Affinitätsliganden bindet, wobei die Proteinvariation die Bindung des Antikörpers an seinen Liganden nicht beeinflusst und der Affinitätsliganden ein Antigen ist;

   (ii) ein Fc-Fusionsprotein ist, das eine Fc-Domäne und eine Effektordomäne umfasst, und die Effektordomäne an den auf dem festen Träger immobilisierten Affinitätsliganden bindet, wobei der Affinitätsligand ein Bindungspartner oder ein Antikörper ist, der spezifisch an die Effektordomäne des Fc-Fusionsproteins bindet; oder

   (iii) ein Fc-Fusionsprotein oder ein Antikörper ist und der Affinitätsligand an die Fc-Domäne des rekombinanten Proteins von Interesse, nicht aber an endogene Antikörper in der Probe bindet.

8. Verfahren nach Anspruch 6 oder 7, wobei das rekombinante Protein von Interesse ein IgG-Antikörper ist, vorzugsweise ein IgG1- oder IgG2-Antikörper, bevorzugter ein humaner oder humanisierter IgG1- oder IgG2-Antikörper.

9. Verfahren nach einem der Ansprüche 3 bis 8, wobei:

(i) das analytische Trennverfahren MS ist, vorzugsweise gebunden an HPLC oder CE, stärker bevorzugt LC-MS und noch stärker bevorzugt NanoLC-MS;

(ii) der feste Träger ein Harz ist, das Mikrokügelchen, vorzugsweise Sepharosekügelchen, Agarosekügelchen oder Magnetkügelchen umfasst, wobei Magnetkügelchen am stärksten bevorzugt sind;

(iii) der Affinitätsligand über N-Hydroxysuccinimid (NHS), Cyanogenbromid, Epoxy, Carbodiimid oder Thiopropyl, vorzugsweise über N-Hydroxysuccinimid (NHS), an den festen Träger gebunden wird; und/oder

(iv) die zwei oder mehreren flüssigen Proben eines Säugetiers zur Analyse in einer Multiwell-Platte, vorzugsweise in einer 24, 96 oder 384 Well-Platte, noch besser in einer 96 Well-Platte, vorzugsweise in einer Multiwell-Filterplatte, vorbereitet werden.

10. Verfahren nach einem der vorhergehenden Ansprüche, wobei die flüssigen Proben eines Säugetiers Körperflüssigkeiten sind, vorzugsweise ausgewählt aus der Gruppe bestehend aus Serum, Plasma, Urin, zerebraler Rückenmarksflüssigkeit, Fruchtwasser, Speichel, Schweiß, Ejakulat, Tränen, Schleim, Vaginalsekret, Vaginalwäsche und Darmwäsche; wobei vorzugsweise die Proben Plasma- oder Serumproben sind.

11. Verfahren nach einem der vorhergehenden Ansprüche, wobei ein oder mehrere pharmakokinetische Parameter mindestens einer spezifischen Proteinvariante des rekombinanten Proteins von Interesse bestimmt werden, vorzugsweise $C_{max}$, $t_{max}$, AUC oder $t_{1/2}$.

12. Verfahren nach einem der vorhergehenden Ansprüche, wobei die Proteinvarianten in einem Aliquot jeder der beiden oder mehreren flüssigen Proben eines Säugetiers analysiert werden und optional die Konzentration des rekombinanten Proteins von Interesse in einem weiteren Aliquot der zwei oder mehreren flüssigen Proben eines Säugers analysiert wird.

13. Verfahren nach einem der vorhergehenden Ansprüche, wobei die Proben oder die zu analysierenden Aliquote ein Volumen von etwa 1 μl bis etwa 1000 μl, von etwa 5 μl bis etwa 500 μl, von etwa 10 μl bis etwa 200 μl, von etwa 10 μl bis etwa 100 μl, von etwa 25 μl bis etwa 100 μl oder von etwa 40 μl bis etwa 75 μl, vorzugsweise von etwa 50 μl, aufweisen.

14. Verfahren nach einem der vorhergehenden Ansprüche, wobei die flüssige Säugetierprobe

(i) von einem Menschen, einem Affen, einem Nagetier, einem Hund, einer Katze oder einem Schwein stammt; oder

(ii) eine nicht-menschliche Probe ist, vorzugsweise eine Probe von Affe, Nagetier, Hund, Katze oder Schwein;

wobei eine Nagetierprobe vorzugsweise von einer Maus, einer Ratte, einem Hamster oder einem Kaninchen stammt.

**Revendications**

1. Procédé d'analyse de variants protéiques d'une protéine recombinante d'intérêt dans des échantillons liquides d'un mammifère, comprenant à

a) fournir deux ou plusieurs échantillons liquides d'un mammifère comprenant la protéine recombinante d'intérêt ;

b) immobiliser la protéine recombinante d'intérêt de chacun desdits échantillons sur un support solide distinct couplé à un ligand par affinité spécifique de la protéine recombinante d'intérêt dans les échantillons ;

c) éluer la protéine recombinante d'intérêt ou l'un de ses fragments de chacun desdits échantillons à partir du support solide dans des éluats séparés ; et à

d) analyser séparément les variants de protéine de l'étape c) de chacun desdits échantillons en utilisant un procédé de séparation analytique et comparer lesdits deux échantillons ou plus, dans lequel un étalon interne se liant au même ligand d'affinité est ajouté à chacun desdits échantillons avant l'étape b) et l'étalon interne est analysé en même temps que la protéine recombinante d'intérêt de l'étape d), et dans lequel les variants protéiques de la protéine recombinante d'intérêt à analyser sont dus à la désamidation, à l'oxydation, à l'hétérogénéité N-terminale, à l'hétérogénéité C-terminale, à l'isomérisation ou aux isoformes de disulfure.

2. Procédé selon la revendication 1, dans lequel le procédé consiste à analyser un ou plusieurs paramètres pharmacocinétiques desdits variants protéiques.

**3.** Procédé selon la revendication 2, dans lequel le procédé de séparation analytique convient pour distinguer lesdites variantes protéiques de la protéine recombinante d'intérêt, de préférence choisies dans le groupe constitué par la HPLC, l'électrophorèse capillaire (CE) ou la spectrométrie de masse (MS).

**4.** Procédé selon la revendication 2, dans lequel

(i) les variants protéiques de la protéine recombinante d'intérêt sont dus à la désamidation, à l'oxydation, à l'hétérogénéité N-terminale, à l'hétérogénéité C-terminale, ou à l'isomérisation, et le procédé de séparation analytique est choisi dans le groupe constitué par HPLC, CE et MS ; ou

(ii) les variants protéiques de la protéine recombinante d'intérêt sont dus à des isoformes de disulfure et le procédé de séparation analytique est choisi dans le groupe constitué de HPLC et de CE.

**5.** Procédé selon l'une quelconque des revendications précédentes, dans lequel l'étalon interne se lie au ligand d'affinité avec une affinité comparable à la protéine recombinante d'intérêt et peut être distingué de la protéine recombinante d'intérêt en utilisant ledit procédé de séparation analytique.

**6.** Procédé selon l'une quelconque des revendications précédentes, dans lequel la protéine d'intérêt recombinante est une protéine de fusion ou un anticorps, de préférence une protéine de fusion Fc ou un anticorps.

**7.** Procédé selon la revendication 6, dans lequel la protéine recombinante d'intérêt est

(i) un anticorps, et la région variable de l'anticorps se lie au ligand par affinité immobilisé sur le support solide, la variation de la protéine n'affectant pas la liaison dudit anticorps à son ligand et le ligand par affinité étant un antigène ;

(ii) une protéine de fusion Fc comprenant un domaine Fc et un domaine effecteur, et le domaine effecteur se lie au ligand d'affinité immobilisé sur le support solide, dans lequel le ligand d'affinité est un partenaire de liaison ou un anticorps se liant spécifiquement au domaine effecteur de la protéine de fusion Fc ; ou

(iii) une protéine de fusion Fc ou un anticorps et le ligand d'affinité se lie au domaine Fc de la protéine recombinante d'intérêt, mais pas aux anticorps endogènes de l'échantillon.

**8.** Procédé selon la revendication 6 ou 7, dans lequel la protéine recombinante d'intérêt est un anticorps IgG, de préférence un anticorps IgG1 ou IgG2, de préférence un anticorps humain ou humanisé IgG1 ou IgG2.

**9.** Procédé selon l'une quelconque des revendications 3 à 8, dans lequel

(i) le procédé de séparation analytique est une MS, de préférence couplée à une HPLC ou à une CE, de préférence une LC-MS et encore de préférence une NanoLC-MS ;

(ii) le support solide est une résine comprenant des microbilles, de préférence des perles de sépharose, des perles d'agarose ou des billes magnétiques, encore de préférence des billes magnétiques ;

(iii) le ligand d'affinité est couplé au support solide via le N-hydroxysuccinimide (NHS), le bromure de cyanogène, l'époxy, le carbodiimide ou le thiopropyle, de préférence via le N-hydroxysuccinimide (NHS) ; et/ou

(iv) les deux échantillons liquides ou plus d'un mammifère sont préparés pour l'analyse dans une plaque à puits multiples, de préférence dans une plaque à 24, 96 ou 384 puits, de préférence dans une plaque à 96 puits, de préférence encore une plaque filtrante à puits multiples.

**10.** Procédé selon l'une quelconque des revendications précédentes, dans lequel les échantillons liquides d'un mammifère sont des fluides corporels, choisis de préférence dans le groupe constitué par le sérum, le plasma, l'urine, le liquide céphalo-rachidien, le liquide amniotique, la salive, la sueur, l'éjaculation, les larmes, les mucosités, les sécrétions vaginales, le lavage vaginal et le lavage colique ;
dans lequel lesdits échantillons sont de préférence des échantillons de plasma ou de sérum.

**11.** Procédé selon l'une quelconque des revendications précédentes, dans lequel un ou plusieurs paramètres pharmacocinétiques d'au moins un variant protéique spécifique de la protéine recombinante d'intérêt sont déterminés, de préférence, $C_{max}$, $t_{max}$, AUC ou $t_{1/2}$.

**12.** Procédé selon l'une quelconque des revendications précédentes, dans lequel les variants protéiques sont analysés dans une partie aliquote de chacun desdits deux échantillons liquides ou plus d'un mammifère et, éventuellement, la concentration de ladite protéine recombinante d'intérêt est analysée dans une autre partie aliquote desdits deux ou échantillons liquides ou plus d'un mammifère.

13. Procédé selon l'une quelconque des revendications précédentes, dans lequel les échantillons ou les aliquotes à analyser ont un volume compris entre environ 1 $\mu$l et environ 1 000 $\mu$l, entre environ 5 $\mu$l et environ 500 $\mu$l, entre environ 10 $\mu$l et environ 200 $\mu$l, entre environ 10 $\mu$l et environ 100 $\mu$l, entre environ 25 $\mu$l et environ 100 $\mu$l, ou entre environ 40 $\mu$l et environ 75 $\mu$l, de préférence d'environ 50 $\mu$l.

14. Procédé selon l'une quelconque des revendications précédentes, dans lequel l'échantillon liquide de mammifère est

(i) un échantillon humain, de singe, de rongeur, de chien, de chat ou de porc ; ou
(ii) un échantillon non humain, de préférence de singe, de rongeur, de chien, de chat ou de porc ;

dans lequel un échantillon de rongeur est de préférence un échantillon de souris, de rat, de hamster ou de lapin.

**Figure 1**

**Figure 2**

Figure 3

**Figure 4**

Figure 5

Figure 6

**Figure 7a**

**Figure 7b**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2975401 A
- WO 2004108948 A

**Non-patent literature cited in the description**

- **ALESSANDRI et al.** *mAbs,* 2012, vol. 4 (4), 509-520
- **CHEN et al.** *Glycobiology,* 2009, vol. 19 (3), 240-249
- **GOETZE et al.** *Glycobiology,* 2011, vol. 21 (7), 949-959
- **HIGEL et al.** *Pharmaceutical Research,* 2015, vol. 32 (11), 3649-59
- **KHAWALI et al.** *mAbs,* 2010, vol. 2 (6), 613-624
- **LIU et al.** *JBC,* 2008, vol. 283 (43), 29266-29272
- **WARD et al.** *Nature,* 1989, vol. 341, 544-546
- **BIRD et al.** *Science,* 1988, vol. 242, 423-426
- **HUSTON et al.** *Proc. Natl. Acad. Sci. USA,* 1988, vol. 85, 5879-5883
- Essentials of Glycobiology. Cold Spring Harbor Laboratory Press, 2009
- **HUBBARD ; IVATT.** *Ann. Rev. Biochem.,* 1981, vol. 50, 555-583
- **DILLON et al.** *JBC,* 2008, vol. 283 (23), 16206-16215
- **LEHTO ; HOU.** Chemistry and Analysis of Radionuclides. Wiley-VCH Verlag & Co, 2011, 170
- **CHERVET et al.** *Analytical Chemistry,* 1996, vol. 68, 1507-12
- Protein Liquid Chromatography. Journal of Chromatography Library. Elsevier Science B.V, 2000, vol. 61, 153
- **MELMER et al.** *Journal of Chromatography A,* 2011, vol. 1218 (1), 118-123
- **MELMER et al.** *Anal Bioanal Chem,* 2010, vol. 398, 905-914